(19) Europäisches Patentamt
European Patent Office
Office européen des brevets

(11) **EP 4 332 983 B1**

(12) **EUROPÄISCHE PATENTSCHRIFT**

(45) Veröffentlichungstag und Bekanntmachung des Hinweises auf die Patenterteilung:
**05.03.2025 Patentblatt 2025/10**

(21) Anmeldenummer: **22192907.8**

(22) Anmeldetag: **30.08.2022**

(51) Internationale Patentklassifikation (IPC):
**G16H 30/40** (2018.01)     **G16H 50/20** (2018.01)
**G16H 50/70** (2018.01)

(52) Gemeinsame Patentklassifikation (CPC):
**G16H 50/70; G16H 30/40; G16H 50/20**

(54) **ERZEUGEN VON SYNTHETISCHEN RADIOLOGISCHEN AUFNAHMEN**

GENERATION OF SYNTHETIC RADIOLOGICAL RECORDINGS

PRODUCTION D'ENREGISTREMENTS RADIOLOGIQUES SYNTHÉTIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**06.03.2024 Patentblatt 2024/10**

(60) Teilanmeldung:
**23163650.7 / 4 336 513**

(73) Patentinhaber: **Bayer Aktiengesellschaft**
**51373 Leverkusen (DE)**

(72) Erfinder:
• **LENGA, Matthias**
**24114, Kiel (DE)**
• **BALTRUSCHAT, Ivo Matteo**
**22299 Hamburg (DE)**
• **KREIS, Felix Karl**
**10249 Berlin (DE)**

(74) Vertreter: **BIP Patents**
**c/o Bayer Intellectual Property GmbH**
**Alfred-Nobel-Straße 50**
**40789 Monheim am Rhein (DE)**

(56) Entgegenhaltungen:
**WO-A1-2021/197996     US-B2- 10 997 716**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Offenbarung bezieht sich auf das technische Gebiet der Radiologie. Gegenstände der vorliegenden Offenbarung sind ein neuartiger Ansatz zum Trainieren eines Modells des maschinellen Lernens zur Erzeugung synthetischer radiologischer Aufnahmen auf Basis von gemessenen radiologischen Aufnahmen und die Verwendung des trainierten Modells des maschinellen Lernens zur Erzeugung von synthetischen radiologischen Aufnahmen.

Medizinische Bildgebung ist die Technik und der Prozess der Abbildung des Körperinneren für klinische Analysen und medizinische Eingriffe sowie die visuelle Darstellung der Funktion bestimmter Organe oder Gewebe. Mit Hilfe der medizinischen Bildgebung sollen innere Strukturen sichtbar gemacht werden, die unter der Haut und den Knochen verborgen sind, und Krankheiten diagnostiziert und/oder behandelt werden.

Fortschritte im Bereich der Bildgebung und des maschinellen Lernens haben zu einem raschen Anstieg des potenziellen Einsatzes von künstlicher Intelligenz bei verschiedenen Aufgaben der medizinischen Bildgebung geführt, z. B. bei der Risikobewertung, Erkennung, Diagnose, Prognose und Therapie.

Dabei werden Modelle des maschinellen Lernens u.a. eingesetzt, um radiologische Aufnahmen zu segmentieren, Kontrastverstärkungen in radiologischen Aufnahmen hervorzurufen und/oder um eine radiologische Aufnahme in einer zeitlichen Sequenz von radiologischen Aufnahmen vorherzusagen.

WO2019/074938A1 offenbart beispielsweise ein Verfahren zur Reduzierung der Menge an Kontrastmittel bei der Erzeugung von radiologischen Aufnahmen mit Hilfe eines künstlichen neuronalen Netzes.

US 10,997,716 B2 offenbart ebenfalls ein Verfahren zur Reduzierung der Menge an Kontrastmittel bei der Erzeugung von radiologischen Aufnahmen mit Hilfe eines künstlichen neuronalen Netzes.

WO2021/052896A1 offenbart ein Verfahren, bei dem eine MRT-Aufnahme der Leber eines Patienten während der hepatobiliären Phase nicht messtechnisch erzeugt, sondern auf Basis von MRT-Aufnahmen aus einer oder mehreren vorangegangenen Phasen berechnet (vorhergesagt) wird, um die Aufenthaltsdauer des Patienten im MRT-Scanner zu verkürzen.

WO2021/197996A1 offenbart ein Verfahren, bei dem MRT-Aufnahmen nach der Applikation eines so genannten Blutpool-Kontrastmittels mit Hilfe eines Modells des maschinellen Lernens simuliert werden.

Bei allen genannten Verfahren wird mindestens eine gemessene radiologische Aufnahme eines Untersuchungsbereichs eines Untersuchungsobjekts einem trainierten Modell des maschinellen Lernens zugeführt und das Modell erzeugt eine synthetische radiologische Aufnahme. Sowohl bei der zugeführten radiologischen Aufnahme als auch bei der synthetischen radiologischen Aufnahme handelt es sich um Repräsentationen des Untersuchungsbereichs in einer Ortsraumdarstellung.

Es hat sich gezeigt, dass die synthetischen radiologischen Aufnahmen oftmals Artefakte aufweisen. Oftmals werden insbesondere feine Strukturen nicht richtig wiedergegeben oder es treten Strukturen in Bereichen der synthetischen radiologischen Aufnahme auf, die das repräsentierte Gewebe nicht aufweist.

Diesem und weiteren Problemen widmet sich die vorliegende Offenbarung.

Ein erster Gegenstand der vorliegenden Offenbarung ist ein Verfahren zum Trainieren eines Modells des maschinellen Lernens zur Erzeugung synthetischer radiologischer Aufnahmen auf Basis von gemessenen radiologischen Aufnahmen. Das Trainingsverfahren umfasst die Schritte:

- Empfangen und/oder Bereitstellen von Trainingsdaten, wobei die Trainingsdaten für jedes Untersuchungsobjekt einer Vielzahl von Untersuchungsobjekten einen Satz an Eingabedaten und Zieldaten umfasst,

  wobei jeder Satz

  ○ mindestens eine Eingabe-Repräsentation eines Untersuchungsbereichs des Untersuchungsobjekts in einem ersten Zustand als Eingabedaten und

  ○ eine Ziel-Repräsentation des Untersuchungsbereichs des Untersuchungsobjekts in einem zweiten Zustand sowie eine transformierte Ziel-Repräsentation als Zieldaten umfasst,

  wobei die transformierte Ziel-Repräsentation zumindest einen Teil des Untersuchungsbereichs des Untersuchungsobjekts

  ○ im Frequenzraum repräsentiert, falls die Ziel-Repräsentation den Untersuchungsbereichs des Untersuchungsobjekts im Ortsraum repräsentiert, oder

  ○ im Ortsraum repräsentiert, falls die Ziel-Repräsentation den Untersuchungsbereich des Untersuchungsobjekts im Frequenzraum repräsentiert,

- Trainieren eines Modells des maschinellen Lernens, wobei das Modell des maschinellen Lernens konfiguriert ist, auf Basis mindestens einer Eingabe-Repräsentation und Modellparametern eine synthetische Repräsentation des Untersuchungsbereichs des Untersuchungsobjekts zu erzeugen, wobei die mindestens eine Eingabe-Repräsentation den Untersuchungsbereich vor und/oder nach der Applikation einer ersten Menge eines Kontrastmittels repräsentiert, und die synthetische Repräsentation den Untersuchungsbereich nach der Applikation einer zweiten Menge des Kontrastmittels repräsentiert, wobei die erste Menge verschieden von der zweiten Menge ist,

wobei das Trainieren für jedes Untersuchungsobjekt der Vielzahl an Untersuchungsobjekten umfasst:

      ∘ Zuführen der mindestens einen Eingabe-Repräsentation dem Modell des maschinellen Lernens,

      o Empfangen einer synthetischen Repräsentation des Untersuchungsbereichs des Untersuchungsobjekts von dem Modell des maschinellen Lernens,

      o Erzeugen und/oder Empfangen einer transformierten synthetischen Repräsentation auf Basis und/oder zu der synthetischen Repräsentation, wobei die transformierte synthetische Repräsentation zumindest einen Teil des Untersuchungsbereichs des Untersuchungsobjekts

            ■ im Frequenzraum repräsentiert, falls die synthetische Repräsentation den Untersuchungsbereichs des Untersuchungsobjekts im Ortsraum repräsentiert, oder

            ■ im Ortsraum repräsentiert, falls die synthetische Repräsentation den Untersuchungsbereich des Untersuchungsobjekts im Frequenzraum repräsentiert,

      o Quantifizieren der Abweichungen i) zwischen zumindest einem Teil der synthetischen Repräsentation und zumindest einem Teil der Ziel-Repräsentation und ii) zwischen zumindest einem Teil der transformierten synthetischen Repräsentation und zumindest einem Teil der transformierten Ziel-Repräsentation mittels einer Fehlerfunktion,

      o Modifizieren von Modellparametern im Hinblick auf reduzierte Abweichungen,

- Ausgeben und/oder Speichern des trainierten Modells des maschinellen Lernens und/oder der Modellparameter und/oder Übermitteln des trainierten Modells des maschinellen Lernens und/oder der Modellparameter an ein separates Computersystem.

Ein weiterer Gegenstand der vorliegenden Offenbarung ist ein computer-implementiertes Verfahren (Vorhersageverfahren) zum Erzeugen einer synthetischen radiologischen Aufnahme mit Hilfe des trainierten Modells des maschinellen Lernens. Das Vorhersageverfahren umfasst die Schritte:

- Bereitstellen eines trainieren Modells des maschinellen Lernens,

      • wobei das trainierte Modell des maschinellen Lernens anhand von Trainingsdaten trainiert worden ist, auf Basis mindestens einer Eingabe-Repräsentation eines Untersuchungsbereichs eines Untersuchungsobjekts in einem ersten Zustand eine synthetische Repräsentation des Untersuchungsbereichs in einem zweiten Zustand zu erzeugen,

      • wobei die Trainingsdaten für jedes Untersuchungsobjekt einer Vielzahl von Untersuchungsobjekten i) eine Eingabe-Repräsentation des Untersuchungsbereichs, ii) eine Ziel-Repräsentation und iii) eine transformierte Ziel-Repräsentation umfassen,

      • wobei die transformierte Ziel-Repräsentation zumindest einen Teil des Untersuchungsbereichs des Untersuchungsobjekts im Frequenzraum repräsentiert, falls die Ziel-Repräsentation den Untersuchungsbereich des Untersuchungsobjekts im Ortsraum repräsentiert, oder im Ortsraum repräsentiert, falls die Ziel-Repräsentation den Untersuchungsbereich des Untersuchungsobjekts im Frequenzraum repräsentiert,

      • wobei das Modell des maschinellen Lernens trainiert worden ist, für jedes Untersuchungsobjekt, Abweichungen zwischen i) zumindest einem Teil der synthetischen Repräsentation und zumindest einem Teil der Ziel-Repräsentation und ii) zwischen zumindest einem Teil einer transformierten synthetischen Repräsentation und zumindest einem Teil der transformierten Ziel-Repräsentation zu minimieren,

- Empfangen mindestens einer Eingabe-Repräsentation eines Untersuchungsbereichs eines neuen Untersuchungsobjekts in dem ersten Zustand, wobei die mindestens eine Eingabe-Repräsentation den Untersuchungsbereich vor und/oder nach der Applikation einer ersten Menge eines Kontrastmittels repräsentiert,

- Eingeben der mindestens einen Eingabe-Repräsentation des Untersuchungsbereichs des neuen Untersuchungsobjekts in das trainierte Modell des maschinellen Lernens,

- Empfangen einer synthetischen Repräsentation des Untersuchungsbereichs des neuen Untersuchungsobjekts in dem zweiten Zustand von dem Modell des maschinellen Lernens, wobei die synthetische Repräsentation den Untersuchungsbereich nach der Applikation einer zweiten Menge des Kontrastmittels repräsentiert, wobei die zweite Menge verschieden von der ersten Menge ist,

- Ausgeben und/oder Speichern der synthetischen Repräsentation und/oder Übermitteln der synthetischen Repräsentation an ein separates Computersystem.

Ein weiterer Gegenstand der vorliegenden Offenbarung ist ein Computersystem umfassend

• eine Empfangseinheit,
• eine Steuer- und Recheneinheit und
• eine Ausgabeeinheit,

- wobei die Steuer- und Recheneinheit konfiguriert ist, die Empfangseinheit zu veranlassen, mindestens eine Eingabe-Repräsentation eines Untersuchungsbereichs eines neuen Untersuchungsobjekts in einem ersten Zustand zu empfangen, wobei die mindestens eine Eingabe-Repräsentation den Untersuchungsbereich vor und/oder nach der Applikation einer ersten Menge eines Kontrastmittels repräsentiert,

- wobei die Steuer- und Recheneinheit konfiguriert ist, die empfangene mindestens eine Eingabe-Repräsentation in ein trainiertes Modell des maschinellen Lernens einzugeben,

    o wobei das trainierte Modell des maschinellen Lernens anhand von Trainingsdaten trainiert worden ist, auf Basis mindestens einer Eingabe-Repräsentation eines Untersuchungsbereichs eines Untersuchungsobjekts in einem ersten Zustand eine synthetische Repräsentation des Untersuchungsbereichs in einem zweiten Zustand zu erzeugen,
    o wobei die Trainingsdaten für jedes Untersuchungsobjekt einer Vielzahl von Untersuchungsobjekten i) eine Eingabe-Repräsentation des Untersuchungsbereichs, ii) eine Ziel-Repräsentation und iii) eine transformierte Ziel-Repräsentation umfassen,
    o wobei die transformierte Ziel-Repräsentation zumindest einen Teil des Untersuchungsbereichs des Untersuchungsobjekts im Frequenzraum repräsentiert, falls die Ziel-Repräsentation den Untersuchungsbereichs des Untersuchungsobjekts im Ortsraum repräsentiert, oder im Ortsraum repräsentiert, falls die Ziel-Repräsentation den Untersuchungsbereich des Untersuchungsobjekts im Frequenzraum repräsentiert,
    o wobei das Modell des maschinellen Lernens trainiert worden ist, für jedes Untersuchungsobjekt, Abweichungen zwischen i) zumindest einem Teil der synthetischen Repräsentation und zumindest einem Teil der Ziel-Repräsentation und ii) zwischen zumindest einem Teil einer transformierten synthetischen Repräsentation und zumindest einem Teil der transformierten Ziel-Repräsentation zu minimieren,

- wobei die Steuer- und Recheneinheit konfiguriert ist, von dem Modell des maschinellen Lernens eine synthetische Repräsentation des Untersuchungsbereichs des neuen Untersuchungsobjekts in dem zweiten Zustand zu empfangen, wobei die synthetische Repräsentation den Untersuchungsbereich nach der Applikation einer zweiten Menge des Kontrastmittels repräsentiert, wobei die zweite Menge verschieden von der ersten Menge ist,

- wobei die Steuer- und Recheneinheit konfiguriert ist, die Ausgabeeinheit zu veranlassen, die synthetische Repräsentation auszugeben und/oder zu speichern und/oder an ein separates Computersystem zu übermitteln.

Ein weiterer Gegenstand der vorliegenden Offenbarung ist ein Computerprogrammprodukt umfassend ein Computerprogramm, das in einen Arbeitsspeicher eines Computersystems geladen werden kann und dort das Computersystem dazu veranlasst, folgende Schritte ausführen:

- Empfangen mindestens einer Eingabe-Repräsentation eines Untersuchungsbereichs eines neuen Untersuchungsobjekts in einem ersten Zustand, wobei die mindestens eine Eingabe-Repräsentation den Untersuchungsbereich vor und/oder nach der Applikation einer ersten Menge eines Kontrastmittels repräsentiert,

- Eingeben der mindestens einen Eingabe-Repräsentation des Untersuchungsbereichs des neuen Untersuchungsobjekts in ein trainiertes Modell des maschinellen Lernens,

    o wobei das trainierte Modell des maschinellen Lernens anhand von Trainingsdaten trainiert worden ist, auf Basis mindestens einer Eingabe-Repräsentation eines Untersuchungsbereichs eines Untersuchungsobjekts in einem ersten Zustand eine synthetische Repräsentation des Untersuchungsbereichs in einem zweiten Zustand zu erzeugen,
    o wobei die Trainingsdaten für jedes Untersuchungsobjekt einer Vielzahl von Untersuchungsobjekten i) eine Eingabe-Repräsentation des Untersuchungsbereichs, ii) eine Ziel-Repräsentation und iii) eine transformierte Ziel-Repräsentation umfassen,
o wobei die transformierte Ziel-Repräsentation zumindest einen Teil des Untersuchungsbereichs des Untersuchungsobjekts im Frequenzraum repräsentiert, falls die Ziel-Repräsentation den Untersuchungsbereichs des Untersuchungsobjekts im Ortsraum repräsentiert, oder im Ortsraum repräsentiert, falls die Ziel-Repräsentation den Untersuchungsbereich des Untersuchungsobjekts im Frequenzraum repräsentiert,
o wobei das Modell des maschinellen Lernens trainiert worden ist, für jedes Untersuchungsobjekt, Abweichungen zwischen i) zumindest einem Teil der synthetischen Repräsentation und zumindest einem Teil der Ziel-Repräsentation und ii) zwischen zumindest einem Teil einer transformierten synthetischen Repräsentation und zumindest einem Teil der transformierten Ziel-Repräsentation zu minimieren,

- Empfangen einer synthetischen Repräsentation des Untersuchungsbereichs des neuen Untersuchungsobjekts in dem zweiten Zustand von dem Modell des maschinellen Lernens, wobei die synthetische Repräsentation den Untersuchungsbereich nach der Applikation einer zweiten Menge des Kontrastmittels repräsentiert, wobei die zweite Menge verschieden von der ersten Menge ist,

- Ausgeben und/oder Speicher der synthetischen Repräsentation und/oder Übermitteln der synthetischen Repräsentation an ein separates Computersystem.

Die Erfindung wird nachstehend näher erläutert, ohne zwischen den Erfindungsgegenständen (Trainingsverfahren, Vorhersageverfahren, Computersystem, Computerprogrammprodukt) zu unterscheiden. Die nachfolgenden Erläuterungen sollen vielmehr für alle Erfindungsgegenstände in analoger Weise gelten, unabhängig davon, in welchem Kontext (Verfahren, Computersystem, Computerprogrammprodukt) sie erfolgen.

Wenn in der vorliegenden Beschreibung oder in den Patentansprüchen Schritte in einer Reihenfolge genannt sind, bedeutet dies nicht zwingend, dass die Erfindung auf die genannte Reihenfolge beschränkt ist. Vielmehr ist denkbar, dass die Schritte auch in einer anderen Reihenfolge oder auch parallel zueinander ausgeführt werden; es sei denn, ein Schritt baut auf einem anderen Schritt auf, was zwingend erforderlich macht, dass der aufbauende Schritt nachfolgend ausgeführt wird (was im Einzelfall aber deutlich wird). Die genannten Reihenfolgen stellen damit bevorzugte Ausführungsformen der Erfindung dar.

Mit Hilfe der vorliegenden Erfindung können Repräsentationen eines Untersuchungsbereichs eines Untersuchungsobjekts vorhergesagt werden. Eine solche vorhergesagte Repräsentation wird in dieser Offenbarung auch als synthetische Repräsentation bezeichnet.

Das "Untersuchungsobjekt" ist üblicherweise ein Lebewesen, vorzugsweise ein Säugetier, ganz besonders bevorzugt ein Mensch.

Der "Untersuchungsbereich" ist ein Teil des Untersuchungsobjekts, zum Beispiel ein Organ oder ein Teil eines Organs wie beispielsweise die Leber, das Gehirn, das Herz, die Niere, die Lunge, der Magen, der Darm, die Bauchspeicheldrüse oder ein Teil der genannten Organe oder mehrere Organe oder ein anderer Teil des Körpers.

In einer bevorzugten Ausführungsform der vorliegenden Erfindung ist der Untersuchungsbereich die Leber oder ein Teil der Leber eines Menschen.

Der Untersuchungsbereich, auch Aufnahmevolumen (engl.: *field of view,* FOV) genannt, stellt insbesondere ein Volumen dar, welches in radiologischen Aufnahmen abgebildet wird. Der Untersuchungsbereich wird typischerweise durch einen Radiologen, beispielsweise auf einer Übersichtsaufnahme (engl.: *localizer*) festgelegt. Selbstverständlich kann der Untersuchungsbereich alternativ oder zusätzlich auch automatisch, beispielsweise auf Grundlage eines ausgewählten Protokolls, festgelegt werden.

Eine "Repräsentation des Untersuchungsbereichs" ist üblicherweise das Ergebnis einer radiologischen Untersuchung.

Die "Radiologie" ist das Teilgebiet der Medizin, das sich mit der Anwendung vorwiegend elektromagnetischer Strahlen und (unter Einbezug etwa der Ultraschalldiagnostik) mechanischer Wellen zu diagnostischen, therapeutischen und/oder wissenschaftlichen Zwecken befasst. Neben Röntgenstrahlen kommen auch andere ionisierende Strahlungen wie Gammastrahlung oder Elektronen zum Einsatz. Da ein wesentlicher Einsatzzweck die Bildgebung ist, werden auch andere bildgebende Verfahren wie die Sonografie und die Magnetresonanztomographie (Kernspintomographie) zur Radiologie gerechnet, obwohl bei diesen Verfahren keine ionisierende Strahlung zum Einsatz kommt. Der Begriff "Radiologie" im Sinne der vorliegenden Erfindung umfasst damit insbesondere die folgenden Untersuchungsmethoden: Computertomografie, Magnetresonanztomografie, Sonografie.

In einer bevorzugten Ausführungsform der vorliegenden Erfindung handelt es sich bei der radiologischen Untersuchung um eine magnetresonanztomographische oder computertomografische Untersuchung. Ganz besonders bevorzugt handelt es sich bei der radiologischen Untersuchung um eine magnetresonanztomographische Untersuchung.

Die Magnetresonanztomographie, abgekürzt MRT oder MRI (engl. MRI: *Magnetic Resonance Imaging*), ist ein bildgebendes Verfahren, das vor allem in der medizinischen Diagnostik zur Darstellung von Struktur und Funktion der Gewebe und Organe im menschlichen oder tierischen Körper eingesetzt wird.

Bei der MR-Bildgebung werden die magnetischen Momente von Protonen in einem Untersuchungsobjekt in einem Grundmagnetfeld ausgerichtet, so dass sich eine makroskopische Magnetisierung entlang einer Längsrichtung einstellt. Diese wird anschließend durch das Einstrahlen von Hochfrequenz(HF)-Pulsen aus der Ruhelage ausgelenkt (Anregung). Die Rückkehr der angeregten Zustände in die Ruhelage (Relaxation) bzw. die Magnetisierungsdynamik wird anschließend mittels einer oder mehrerer HF-Empfangsspulen als Relaxationssignale detektiert.

Zur Ortskodierung werden dem Grundmagnetfeld schnell geschaltete magnetische Gradientenfelder überlagert. Die erfassten Relaxationssignale bzw. die detektierten MR-Daten liegen zunächst als Rohdaten im Frequenzraum vor (so genannte k-Raum-Daten), und können durch anschließende inverse Fourier-Transformation in den Ortsraum (Bildraum) transformiert werden.

Eine Repräsentation des Untersuchungsbereichs im Sinne der vorliegenden Offenbarung kann eine MRT-Aufnahme, ein Computertomogramm, ein Ultraschallbild oder dergleichen sein oder die Repräsentation des Untersuchungsbereichs kann aus einer oder mehreren MRT-Aufnahmen, Computertomogrammen, Ultraschallbildern oder dergleichen erzeugt werden.

Eine Repräsentation des Untersuchungsbereichs im Sinne der vorliegenden Offenbarung kann eine Repräsentation im Ortsraum (Bildraum) oder eine Repräsentation im Frequenzraum sein.

In einer Repräsentation im Ortsraum, in dieser Beschreibung auch als Ortsraumdarstellung oder Ortsraum-Repräsentation bezeichnet, wird der Untersuchungsbereich üblicherweise durch eine Vielzahl an Bildelementen (Pixeln oder Voxel) repräsentiert, die beispielsweise rasterförmig angeordnet sein können, und wobei jedes Bildelement einen Teil des Untersuchungsbereichs repräsentiert. Ein in der Radiologie weit verbreitetes Format zur Speicherung und Verarbeitung von Repräsentationen im Ortsraum ist das DICOM-Format. DICOM (Digital Imaging and Communications in Medicine) ist ein offener Standard zur Speicherung und zum Austausch von Informationen im medizinischen Bilddatenmanagement.

In einer Repräsentation im Frequenzraum, in dieser Beschreibung auch als Frequenzraumdarstellung oder Frequenzraum-Repräsentation bezeichnet, wird der Untersuchungsbereich durch eine Überlagerung von Grundschwingungen repräsentiert. Beispielsweise kann der Untersuchungsbereich durch eine Summe von Sinus- und Kosinus-Funktionen mit verschiedenen Amplituden, Frequenzen und Phasen repräsentiert werden. Die Amplituden und Phasen können als Funktion der Frequenzen beispielsweise in einer zwei- oder dreidimensionalen Darstellung aufgetragen werden. Üblicherweise wird die niedrigste Frequenz (Ursprung) in das Zentrum gelegt. Je weiter man sich von diesem Zentrum entfernt, desto höher sind die Frequenzen. Jeder Frequenz kann eine Amplitude, mit der die Frequenz in der Frequenzraumdarstellung vertreten ist, und eine Phase, die angibt, inwieweit die jeweilige Schwingung gegenüber einer Sinus- oder Kosinus-Schwingung verschoben ist, zugeordnet werden.

Eine Repräsentation im Ortsraum lässt sich beispielsweise durch eine Fourier-Transformation in eine Repräsentation im Frequenzraum überführen (transformieren). Umgekehrt lässt sich eine Repräsentation im Frequenzraum beispielsweise durch eine inverse Fourier-Transformation in eine Repräsentation im Ortsraum überführen (transformieren).

Details über Ortsraumdarstellungen und Frequenzraumdarstellungen und ihre jeweilige Umwandlung ineinander sind in zahlreichen Publikationen beschrieben, siehe z.B.: https://see.stanford.edu/materials/1softaee261/book-fall-07.pdf.

Mit Hilfe eines trainierten Modells des maschinellen Lernens kann auf Basis mindestens einer Repräsentation eines Untersuchungsbereichs eines Untersuchungsobjekts in einem ersten Zustand eine synthetische Repräsentation des Untersuchungsbereichs des Untersuchungsobjekts in einem zweiten Zustand erzeugt werden. Die mindestens eine Repräsentation, auf deren Basis das (trainierte) Modell des maschinellen Lernens die synthetische Repräsentation erzeugt, wird in dieser Beschreibung auch als Eingabe-Repräsentation bezeichnet.

Die synthetische Repräsentation repräsentiert üblicherweise denselben Untersuchungsbereich desselben Untersuchungsobjekts wie die mindestens eine Eingabe-Repräsentation, auf deren Erzeugung die synthetische Repräsentation basiert. Die Eingabe-Repräsentation repräsentiert den Untersuchungsbereich in einem ersten Zustand; die synthetische Repräsentation repräsentiert den Untersuchungsbereich in einem zweiten Zustand. Der erste Zustand und der zweite Zustand sind unterschiedliche Zustände.

Die synthetische Repräsentation repräsentiert den Untersuchungsbereich mit einer anderen Menge an Kontrastmittel als die Eingabe-Repräsentation.

In einer bevorzugten Ausführungsform ist das Modell des maschinellen Lernens konfiguriert und trainiert, auf Basis mindestens einer Eingabe-Repräsentation eines Untersuchungsbereichs eines Untersuchungsobjekts, die den Untersuchungsbereich vor und/oder nach der Applikation einer ersten Menge an Kontrastmittel repräsentiert, eine synthetische Repräsentation zu erzeugen, die den Untersuchungsbereich nach der Applikation einer zweiten Menge an Kontrastmittel repräsentiert, wobei die erste Menge geringer als die zweite Menge ist. Ein solches trainiertes Modell des maschinellen Lernens kann beispielsweise wie in WO2019/074938A1 beschrieben eingesetzt werden, um die Menge an Kontrastmittel bei radiologischen Untersuchungen zu reduzieren. Ein solches trainiertes Modell des maschinellen Lernens kann eingesetzt werden, um eine radiologische Aufnahme, die nach der Applikation einer ersten (geringeren) Menge eines Kontrastmittels erzeugt worden ist, in eine radiologische Aufnahme umzuwandeln, die in Bezug auf die Kontrastverteilung so aussieht wie eine radiologische Aufnahme nach der Applikation einer zweiten (größeren) Menge des Kontrastmittels. Mit anderen Worten, das Modell des maschinellen Lernens kann trainiert werden, eine Kontrastverstärkung zu erzeugen, ohne dass die Menge an Kontrastmittel erhöht werden muss.

Ein "Modell des maschinellen Lernens" kann als eine computerimplementierte Datenverarbeitungsarchitektur verstanden werden. Das Modell kann Eingabedaten empfangen und Ausgabedaten auf der Grundlage dieser Eingabedaten und Modellparametern liefern. Das Modell kann durch Training eine Beziehung zwischen den Eingabedaten und den Ausgabedaten erlernen. Beim Training können Modellparameter angepasst werden, um eine gewünschte Ausgabe für eine bestimmte Eingabe zu liefern.

Beim Trainieren eines solchen Modells werden dem Modell Trainingsdaten präsentiert, aus denen es lernen kann. Das trainierte Modell des maschinellen Lernens ist das Ergebnis des Trainingsprozesses. Die Trainingsdaten umfassen neben Eingabedaten die korrekten Ausgabedaten (Zieldaten), die das Modell auf Basis der Eingabedaten erzeugen soll. Beim Trainieren werden Muster erkannt, die die Eingabedaten auf die Zieldaten abbilden.

Im Trainingsprozess werden die Eingabedaten der Trainingsdaten in das Modell eingegeben, und das Modell erzeugt Ausgabedaten. Die Ausgabedaten werden mit den Zieldaten verglichen. Modellparameter werden so verändert, dass die Abweichungen zwischen den Ausgabedaten und den Zieldaten auf ein (definiertes) Minimum reduziert werden. Zur Modifizierung der Modellparameter im Hinblick auf eine Reduzierung der Abweichungen kann ein Optimierungsverfahren wie beispielsweise ein Gradientenverfahren verwendet werden.

Die Abweichungen können mit Hilfe einer Fehlerfunktion (engl.: *loss function*) quantifiziert werden. Eine solche Fehlerfunktion kann verwendet werden, um einen Fehler (engl.: *loss*) für ein gegebenes Paar von Ausgabedaten und Zieldaten zu berechnen. Das Ziel des Trainingsprozesses kann darin bestehen, die Parameter des Modells des maschinellen Lernens so zu verändern (anzupassen), dass der Fehler für alle Paare des Trainingsdatensatzes auf ein (definiertes) Minimum reduziert wird.

Handelt es sich bei den Ausgabedaten und den Zieldaten beispielsweise um Zahlen, kann die Fehlerfunktion die absolute Differenz zwischen diesen Zahlen sein. In diesem Fall kann ein hoher absoluter Fehler bedeuten, dass ein oder mehrere Modellparameter in hohem Maße geändert werden müssen.

Bei Ausgabedaten in Form von Vektoren können beispielsweise Differenzmetriken zwischen Vektoren wie der mittlere quadratische Fehler, ein Kosinusabstand, eine Norm des Differenzvektors wie ein euklidischer Abstand, ein Tschebyscheff-Abstand, eine Lp-Norm eines Differenzvektors, eine gewichtete Norm oder eine andere Art von Differenzmetrik zweier Vektoren als Fehlerfunktion gewählt werden.

Bei höherdimensionalen Ausgaben, wie z.B. zweidimensionalen, dreidimensionalen oder höherdimensionalen Ausgaben, kann z.B. eine elementweise Differenzmetrik verwendet werden. Alternativ oder zusätzlich können die Ausgabedaten vor der Berechnung eines Fehlerwertes transformiert werden, z.B. in einen eindimensionalen Vektor.

Im vorliegenden Fall wird das Modell des maschinellen Lernens anhand von Trainingsdaten trainiert, eine synthetische Repräsentation eines Untersuchungsbereichs eines Untersuchungsobjekts in einem zweiten Zustand auf Basis von mindestens einer Repräsentation des Untersuchungsbereichs des Untersuchungsobjekts in einem ersten Zustand zu erzeugen.

Die Trainingsdaten umfassen für jedes Untersuchungsobjekt einer Vielzahl von Untersuchungsobjekten einen Satz an Eingabedaten und Zieldaten.

Der Begriff Vielzahl bedeutet mindestens zehn, vorzugsweise mehr als einhundert.

Jeder Satz an Eingabedaten und Zieldaten umfasst mindestens eine Eingabe-Repräsentation eines Untersuchungsbereich des Untersuchungsobjekts in einem ersten Zustand als Eingabedaten. Jeder Satz an Eingabedaten und Zieldaten umfasst ferner eine Ziel-Repräsentation des Untersuchungsbereichs des Untersuchungsobjekts in einem zweiten Zustand und eine transformierte Ziel-Repräsentation als Zieldaten.

Der Untersuchungsbereich ist üblicherweise für alle Untersuchungsobjekte der gleiche.

Die transformierte Ziel-Repräsentation repräsentiert zumindest einen Teil des Untersuchungsbereichs des Untersuchungsobjekts in dem zweiten Zustand.

Für den Fall, dass die Ziel-Repräsentation den Untersuchungsbereich des Untersuchungsobjekts im Ortsraum repräsentiert, repräsentiert die transformierte Ziel-Repräsentation zumindest einen Teil des Untersuchungsbereichs des Untersuchungsobjekts im Frequenzraum.

Für den Fall, dass die Ziel-Repräsentation den Untersuchungsbereich des Untersuchungsobjekts im Frequenzraum repräsentiert, repräsentiert die transformierte Ziel-Repräsentation zumindest einen Teil des Untersuchungsbereichs des Untersuchungsobjekts im Ortsraum.

Die transformierte Ziel-Repräsentation im Frequenzraum kann aus einer Ziel-Repräsentation im Ortsraum beispielsweise durch Fourier-Transformation erzeugt werden.

Die transformierte Ziel-Repräsentation im Ortsraum kann aus einer Ziel-Repräsentation im Frequenzraum beispielsweise durch inverse Fourier-Transformation erzeugt werden.

Beim Trainieren des Modells des maschinellen Lernens wird für jedes Untersuchungsobjekt der Vielzahl an Untersuchungsobjekten die mindestens eine Eingabe-Repräsentation des Untersuchungsbereichs dem Modell des maschinellen Lernens zugeführt. Das Modell erzeugt auf Basis der mindestens einen Eingabe-Repräsentation des Untersuchungsbereichs und auf Basis von Modellparametern eine synthetische Repräsentation.

Für den Fall, dass die dem Modell zugeführte mindestens eine Eingabe-Repräsentation den Untersuchungsbereich im Ortsraum repräsentiert, repräsentiert die synthetische Eingabe-Repräsentation den Untersuchungsbereich vorzugsweise (aber nicht notwendigerweise) ebenfalls im Ortsraum.

Für den Fall, dass die dem Modell zugeführte mindestens eine Eingabe-Repräsentation den Untersuchungsbereich im Frequenzraum repräsentiert, repräsentiert die synthetische Repräsentation den Untersuchungsbereich vorzugsweise (aber nicht notwendigerweise) ebenfalls im Frequenzraum.

Das Modell des maschinellen Lernens kann jedoch auch konfiguriert sein und trainiert werden, auf Basis der mindestens einen Eingabe-Repräsentation des Untersuchungsbereichs eine synthetische Repräsentation des Untersuchungsbereichs zu erzeugen, die i) den Untersuchungsbereich im Frequenzraum repräsentiert falls die mindestens eine Repräsentation den Untersuchungsbereich im Ortsraum repräsentiert, oder ii) den Untersuchungsbereich im Ortsraum repräsentiert falls die mindestens eine Repräsentation den Untersuchungsbereich im Frequenzraum repräsentiert. Mit anderen Worten: das Modell des maschinellen Lernens kann konfiguriert sein und trainiert werden, (unter anderem) eine Transformation vom Orts- in den Frequenzraum oder umgekehrt auszuführen.

Von oder zu der synthetischen Repräsentation wird eine transformierte synthetische Repräsentation erzeugt. Für den Fall, dass die synthetische Repräsentation den Untersuchungsbereich im Ortsraum in einem zweite Zustand repräsentiert, repräsentiert die transformierte synthetische Repräsentation zumindest einen Teil des Untersuchungsbereichs in dem zweiten Zustand im Frequenzraum. Für den Fall, dass die synthetische Repräsentation den Untersuchungsbereich in dem zweiten Zustand im Frequenzraum repräsentiert, repräsentiert die transformierte synthetische Repräsentation zumindest einen Teil des Untersuchungsbereichs in dem zweiten Zustand im Ortsraum. Die Erzeugung der transformierten synthetischen Repräsentation kann durch Transformation der synthetischen Repräsentation erfolgen und/oder das Modell des maschinellen Lernens kann konfiguriert sein und trainiert werden, eine transformierte synthetische Repräsentation auf Basis der mindestens einen Eingabe-Repräsentation zu erzeugen.

Mittels einer Fehlerfunktion werden die Abweichungen i) zwischen zumindest einem Teil der synthetischen Repräsentation und zumindest einem Teil der Zeil-Repräsentation und ii) zwischen zumindest einem Teil der transformierten synthetischen Repräsentation und zumindest einem Teil der transformierten Ziel-Repräsentation quantifiziert.

Die Fehlerfunktion kann zwei Terme aufweisen, einen ersten Term zur Quantifizierung der Abweichungen zwischen zumindest einem Teil der synthetischen Repräsentation und zumindest einem Teil der Ziel-Repräsentation und einen zweiten Term zur Quantifizierung der Abweichungen zwischen zumindest einem Teil der transformierten synthetischen Repräsentation und zumindest einem Teil der transformierten Ziel-Repräsentation. Die Terme können in der Fehlerfunktion beispielsweise addiert werden. Die Terme können in der Fehlerfunktion unterschiedlich gewichtet werden. Die nachfolgende Gleichung gibt ein Beispiel für eine (Gesamt-) Fehlerfunktion $L$ zur Quantifizierung der Abweichungen an:

$$L = \lambda_1 \cdot L_1 + \lambda_2 \cdot L_2 \quad (\text{Gl. 1})$$

Dabei ist $L$ die (Gesamt-)Fehlerfunktion, $L_1$ ein Term, der die Abweichungen zwischen der synthetischen Repräsentation und der Ziel-Repräsentation repräsentiert, $L_2$ ein Term, der die Abweichungen zwischen der transformierten synthetischen Repräsentation und der transformierten Ziel-Repräsentation quantifiziert, und $\lambda_1$ und $\lambda_2$ Gewichtsfaktoren, die beispielsweise Werte zwischen 0 und 1 annehmen können und den beiden Termen ein unterschiedliches Gewicht in der Fehlerfunktion geben. Es ist möglich, dass die Gewichtsfaktoren während des Trainierens des Modells des maschinellen Lernens konstant gehalten werden oder variiert werden.

Beispiele für Fehlerfunktionen, die allgemein zur Ausführung der vorliegenden Erfindung verwendet werden können, sind L1-Fehlerfunktion (*L1 loss*), L2-Fehlerfunktion (*L2 loss*), Lp-Fehlerfunktion (Lp loss), Strukturähnlichkeitsindexmaß (*structural similarity index measure* (SSIM)), VGG-Fehlerfunktion (VGG loss), Wahrnehmungs-Fehlerfunktion (*perceptual loss*) oder eine Kombination der oben genannten Funktionen oder andere Fehlerfunktionen. Weitere Einzelheiten zu Fehlerfunktionen sind z.B. in der wissenschaftlichen Literatur zu finden (siehe z. B.: R. Mechrez et al.: The Contextual Loss for Image Transformation with Non-Aligned Data, 2018, arXiv:1803.02077v4; H. Zhao et al.: Loss Functions for Image Restoration with Neural Networks, 2018, arxiv:1511.08861v3).

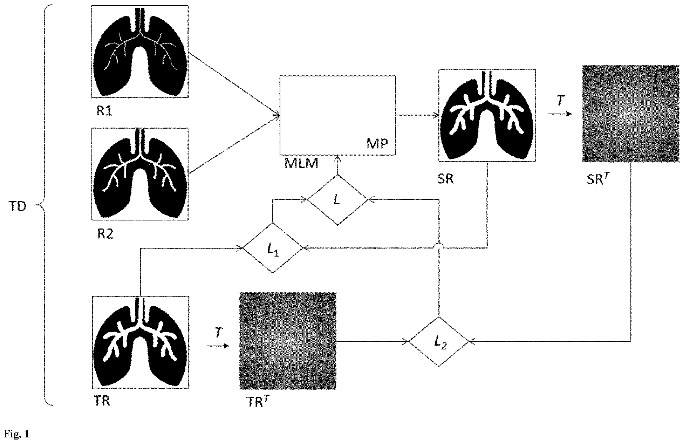

Fig. 1 zeigt beispielhaft und schematisch den Prozess des Trainierens des Modells des maschinellen Lernens. Das Trainieren erfolgt mit Hilfe von Trainingsdaten. In Fig. 1 sind Trainingsdaten TD für ein Untersuchungsobjekt gezeigt. Die Trainingsdaten TD umfassen als Eingabedaten eine erste Eingabe-Repräsentation R1 eines Untersuchungsbereichs des Untersuchungsobjekts und eine zweite Eingabe-Repräsentation R2 des Untersuchungsbereichs des Untersuchungsobjekts. Die erste Eingabe-Repräsentation R1 und die zweite Eingabe-Repräsentation R2 repräsentieren den Untersuchungsbereich im Ortsraum. Die erste Eingabe-Repräsentation R1 repräsentiert den Untersuchungsbereich in einem ersten Zustand, zum Beispiel vor oder nach der Applikation einer ersten Menge eines Kontrastmittels. Die zweite Eingabe-Repräsentation R2 repräsentiert den Untersuchungsbereich in einem zweiten Zustand, zum Beispiel nach der Applikation einer zweiten Menge des Kontrastmittels.

Die Trainingsdaten TD umfassen ferner eine Ziel-Repräsentation TR als Zieldaten. Die ZielRepräsentation TR repräsentiert den Untersuchungsbereich ebenfalls im Ortsraum. Die ZielRepräsentation TR repräsentiert den Untersuchungsbereich in einem dritten Zustand, beispielsweise nach der Applikation einer dritten Menge des Kontrastmittels.

Das Modell des maschinellen Lernens MLM wird trainiert, auf Basis der ersten Eingabe-Repräsentation R1 und der zweiten Eingabe-Repräsentation R2 sowie auf Basis von Modellparametern MP die ZielRepräsentation TR vorherzusagen. Die erste Eingabe-Repräsentation R1 und die zweite Eingabe-Repräsentation R2 werden dem Modell des maschinellen Lernens als Eingabedaten zugeführt. Das Modell des maschinellen Lernens ist konfiguriert, eine synthetische Repräsentation SR zu erzeugen.

Von der synthetischen Repräsentation SR wird in dem in Fig. 1 gezeigten Beispiel mittels einer Transformation T (z.B. einer Fourier-Transformation) eine transformierte synthetische Repräsentation $SR^T$ erzeugt. Analog wird von der Ziel-Repräsentation TR mittels der Transformation T eine transformierte Ziel-Repräsentation $TR^T$ erzeugt. Die transformierte synthetische Repräsentation $SR^T$ und die transformierte Ziel-Repräsentation $TR^T$ sind Frequenzraumdarstellungen des Untersuchungsbereichs im dritten Zustand.

Eine erste Fehlerfunktion $L_1$ wird verwendet, um die Abweichungen zwischen der synthetischen Repräsentation SR und der Ziel-Repräsentation TR zu quantifizieren. Eine zweite Fehlerfunktion $L_2$ wird verwendet, um die Abweichungen zwischen der transformierten synthetischen Repräsentation $SR^T$ und der transformierten Ziel-Repräsentation $TR^T$ zu quantifizieren. Die mittels der Fehlerfunktionen $L_1$ und $L_2$ berechneten Fehler werden in einer Gesamtfehlerfunktion L zu einem Gesamtfehler zusammengefasst (z.B. durch Addition).

Modellparameter MP werden im Hinblick auf eine Reduzierung des Gesamtfehlers modifiziert. Die Reduzierung des Gesamtfehlers kann mit Hilfe eines Optimierungsverfahrens erfolgen, beispielsweise mit Hilfe eines Gradientenverfahrens.

Der Prozess wird für eine Vielzahl an Untersuchungsobjekten wiederholt, bis der Gesamtfehler ein vordefiniertes (gewünschtes) Minimum erreicht hat.

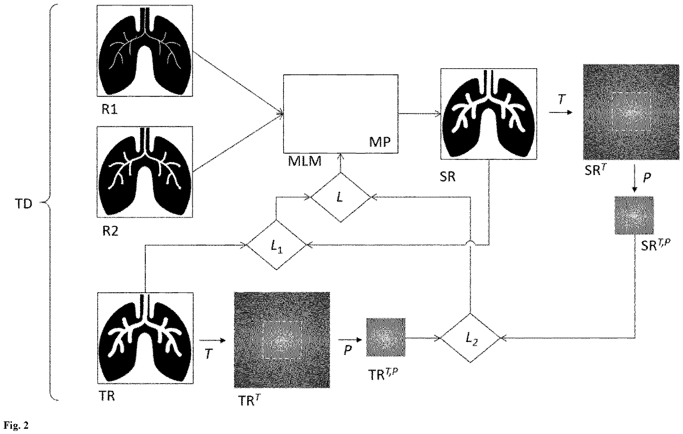

In einer bevorzugten Ausführungsform werden die transformierte synthetische Repräsentation und die transformierte Ziel-Repräsentation jeweils auf einen oder mehrere vordefinierte Anteile reduziert. Dies ist beispielhaft und schematisch in Fig. 2 dargestellt.

Der in Fig. 2 dargestellte Prozess entspricht dem Prozess, der in Fig. 1 dargestellt ist, mit folgenden Unterschieden:

- Die transformierte Ziel-Repräsentation $TR^T$ wird auf einen definierten Anteil $TR^{T,P}$ reduziert. Die Repräsentation $TR^{T,P}$ ist ein Teil der transformierten Ziel-Repräsentation TR. Die Repräsentation $TR^{T,P}$ kann durch eine Funktion P aus der transformierten Ziel-Repräsentation $TR^T$ erzeugt werden, indem beispielsweise alle Werte von Frequenzen außerhalb des vordefinierten Teils auf Null gesetzt werden.
- Analog wird die transformierte synthetische Repräsentation $SR^T$ wird auf einen definierten Anteil $SR^{T,P}$ reduziert.
- Die Anteile, auf die die transformierte Ziel-Repräsentation $TR^T$ und die transformierte synthetische Repräsentation $SR^T$ reduziert werden, korrespondieren üblicherweise miteinander, d.h. sie betreffen üblicherweise dieselben Frequenzen. In dem in Fig. 2 dargestellten Prozess ist sowohl die die transformierte Ziel-Repräsentation $TR^T$ als auch die transformierte synthetische Repräsentation $SR^T$ auf jeweils einen Anteil reduziert worden, der die niedrigen Frequenzen in einem Bereich (z.B. Rechteck oder Quadrat) um das Zentrum herum beinhaltet (siehe die gestrichelten weißen Rahmen). In diesem Bereich sind überwiegend Kontrastinformationen kodiert).

In dem in Fig. 2 dargestellten Beispiel wird also nicht der gesamte Frequenzraum bei der Berechnung mittels der Fehlerfunktion $L_2$ betrachtet. Die Repräsentationen im Frequenzraum werden auf einen Bereich mit niedrigen Frequenzen reduziert; die höheren Frequenzen werden verworfen. In dem Bereich der niedrigen Frequenzen sind überwiegend Kontrastinformationen kodiert, während im Bereich der höheren Frequenzen überwiegend Informationen zu Feinstrukturen kodiert sind. Das bedeutet, dass das Modell des maschinellen Lernens in dem in Fig. 2 dargestellten Beispiel zusätzlich zu der Erzeugung der synthetischen Repräsentation SR trainiert wird, insbesondere die niedrigen Frequenzen korrekt wiederzugeben und damit ein besonderer Fokus auf Kontrastinformationen gelegt wird.

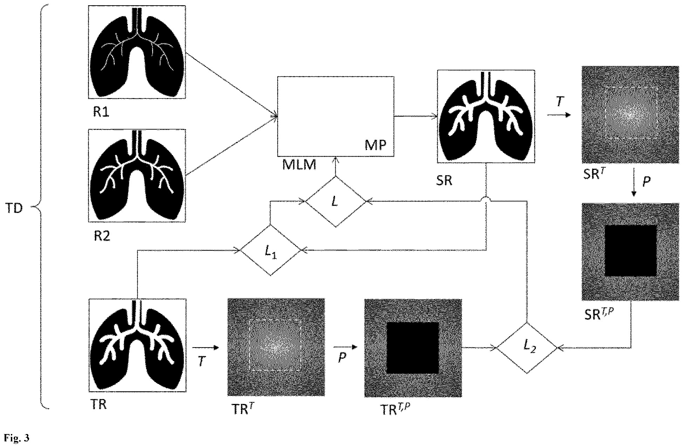

Fig. 3 zeigt ein weiteres Beispiel, bei dem nicht der gesamte Frequenzraum betrachtet, sondern ein Fokus auf einen definierten Frequenzbereich gelegt wird. Der in Fig. 3 dargestellte Prozess entspricht dem Prozess, der in Fig. 2 dargestellt ist, mit dem Unterschied, dass die Funktion P dafür sorgt, dass die transformierte Ziel-Repräsentation $TR^T$ und die transformierte synthetische Repräsentation $SR^T$ jeweils auf einen Anteil mit höheren Frequenzen reduziert werden, während niedrige Frequenzen verworfen werden (die niedrigen Frequenzwerte werden im vorliegenden Beispiel auf den Wert Null gesetzt, der durch die Farbe schwarz repräsentiert wird). Das bedeutet, dass das Modell des maschinellen Lernens in dem in Fig. 3 dargestellten Beispiel zusätzlich zu der Erzeugung der synthetischen Repräsentation SR trainiert wird, insbesondere die höheren Frequenzen korrekt wiederzugeben und damit ein besonderer Fokus auf die korrekte Wiedergabe von Feinstrukturen gelegt wird.

Es sei angemerkt, dass der Teil, auf den der Frequenzraum reduziert wird, auch andere als die in Fig. 2 und Fig. 3 gezeigten Formen und Größen annehmen kann. Ferner können mehrere Teile ausgewählt und die Frequenzraum-Darstellungen auf mehrere Teile (Bereiche) beschränkt werden. So ist es beispielsweise möglich, den Frequenzraum in verschiedene Bereiche zu unterteilen und für mehrere oder alle Bereiche Frequenzraum-Darstellungen des Untersuchungsbereichs zu erzeugen, in denen jeweils nur die Frequenzen des jeweiligen Bereichs auftreten. Auf diese Weise können unterschiedliche Frequenzbereiche unterschiedlich stark beim Trainieren gewichtet/berücksichtigt werden.

In den in Fig. 2 und Fig. 3 gezeigten Beispielen handelt es sich bei den Repräsentationen R1, R2, TR und SR um Repräsentationen im Ortsraum. Es ist ebenso möglich, dass eine oder mehrere dieser Repräsentationen Frequenzraum-Darstellungen sind.

Sind die transformierte synthetische Repräsentation $SR^T$ und die transformierte Ziel-Repräsentation $TR^T$ Repräsentationen im Ortsraum, kann ebenfalls ein Teil (oder mehrere Teile) in den Repräsentationen ausgewählt und die Repräsentationen können auf diesen Teil (diese Teile) reduziert werden. In einem solchen Fall wird also bei der Fehlerberechnung Fokus auf Merkmale im Ortsraum gelegt, die sich in dem ausgewählten Teil (den ausgewählten Teilen) wiederfinden.

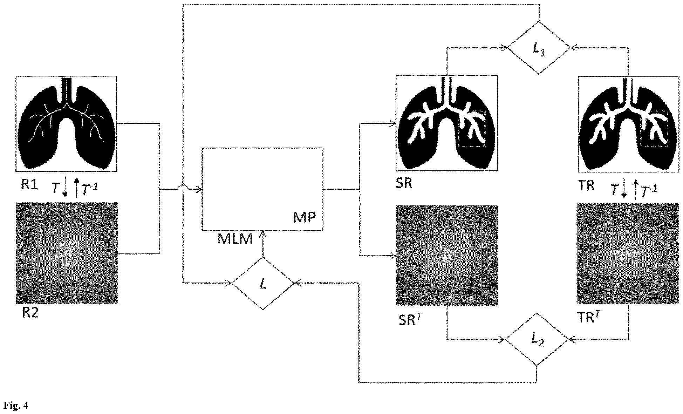

Fig. 4, Fig. 5, Fig. 6, Fig. 7 und Fig. 8 zeigen weitere Ausführungsformen des Trainingsverfahrens.

In der in Fig. 4 gezeigten Ausführungsform umfassen die Trainingsdaten für jedes Untersuchungsobjekt mindestens eine Eingabe-Repräsentation R1 eines Untersuchungsbereichs in einem ersten Zustand und eine Ziel-Repräsentation TR des Untersuchungsbereichs in einem zweiten Zustand. Die mindestens eine Eingabe-Repräsentation R1 und die Ziel-Repräsentation TR können jeweils eine Repräsentation im Ortsraum oder eine Repräsentation im Frequenzraum sein. In dem in Fig. 4 gezeigten Beispiel sind die Eingabe-Repräsentation R1 und die Ziel-Repräsentation TR Repräsentationen im Ortsraum. Das Modell des maschinellen Lernens MLM wird trainiert, die Ziel-Repräsentation TR auf Basis der mindestens einen Eingabe-Repräsentation R1 vorherzusagen. Mittels einer Transformation T wird auf Basis der Eingabe-Repräsentation R1 eine transformierte Eingabe-Repräsentation R2 erzeugt. Analog wird mittels der Transformation T eine transformierte Ziel-Repräsentation $TR^T$ auf Basis der ZielRepräsentation TR erzeugt. In dem in Fig. 4 gezeigten Beispiel sind die transformierte Eingabe-Repräsentation R2 und die transformierte Ziel-Repräsentation $TR^T$ Repräsentationen des Untersuchungsbereichs im Frequenzraum. Die transformierte Eingabe-Repräsentation R2 repräsentiert den Untersuchungsbereich (wie die Eingabe-Repräsentation R1) in dem ersten Zustand; die transformierte Ziel-Repräsentation $TR^T$ repräsentiert den Untersuchungsbereich (wie die ZielRepräsentation TR) in dem zweiten Zustand.

In Fig. 4 ist dargestellt, dass die Eingabe-Repräsentation R1 durch eine inverse Transformation $T^{-1}$ auf Basis der transformierten Eingabe-Repräsentation R2 gewonnen werden kann. Analog kann eine ZielRepräsentation TR durch die inverse Transformation $T^{-1}$ auf Basis der transformierten ZielRepräsentation $TR^T$ gewonnen werden. Die inverse Transformation $T^{-1}$ ist die zu der Transformation T inverse Transformation, was durch den Index -1 gekennzeichnet ist. Der Hinweis auf die inverse Transformation soll verdeutlichen, dass die Trainingsdaten zumindest eine Eingabe-Repräsentation (R1 oder R2) und eine Ziel-Repräsentation (TR oder $TR^T$) enthalten müssen; die jeweils andere (R2 oder R1, bzw. $TR^T$ oder TR) kann durch Transformation oder inverse Transformation aus der vorliegenden Repräsentation gewonnen werden. Dies gilt generell und nicht nur für die in Fig. 4 dargestellte Ausführungsform.

In der in Fig. 4 gezeigten Ausführungsform wird sowohl die Eingabe-Repräsentation R1 als auch die transformierte Eingabe-Repräsentation R2 dem Modell des maschinellen Lernens MLM zugeführt. Das Modell des maschinellen Lernens MLM ist konfiguriert, sowohl eine synthetische Repräsentation SR als auch eine transformierte synthetische Repräsentation $SR^T$ zu erzeugen.

Mittels eine ersten Fehlerfunktion $L_1$ werden die Abweichungen zwischen zumindest einem Teil der synthetischen Repräsentation SR und zumindest einem Teil der Ziel-Repräsentation TR quantifiziert. Mittels einer zweiten Fehlerfunktion $L_2$ werden die Abweichungen zwischen zumindest einem Teil der transformierten synthetischen Repräsentation $SR^T$ und zumindest einem Teil der transformierten ZielRepräsentation $TR^T$ quantifiziert. Die in Fig. 4 in den transformierten Repräsentationen eingezeichneten gestrichelten weißen Rahmen sollen zum Ausdruck bringen, dass die Berechnung des Fehlers mit der Fehlerfunktion wie in Bezug zu Fig. 3 beschrieben nicht auf der gesamten Frequenzraumdarstellung beruhen muss, sondern dass die transformierten Repräsentationen auf einen Frequenzbereich (oder mehrere Frequenzbereiche) reduziert werden können. Analog können die Ortsraumdarstellungen SR und TR bei der Fehlerberechnung auf einen Teil (oder mehrere Teile) reduziert werden (ebenfalls dargestellt durch die gestrichelten weißen Rahmen). Dies gilt analog auch für die übrigen Ausführungsformen und nicht nur für die in Fig. 4 dargestellte Ausführungsform.

Die mittels der Fehlerfunktionen $L_1$ und $L_2$ berechneten Fehler werden in einer Gesamtfehlerfunktion *L* zu einem Gesamtfehler zusammengefasst (z.B. durch Addition).

Modellparameter MP werden im Hinblick auf eine Reduzierung des Gesamtfehlers modifiziert. Dies kann mittels eines Optimierungsverfahrens, beispielsweise eines Gradientenverfahrens erfolgen.

Der Prozess wird für eine Vielzahl an Untersuchungsobjekten wiederholt, bis der Gesamtfehler ein vordefiniertes (gewünschtes) Minimum erreicht hat.

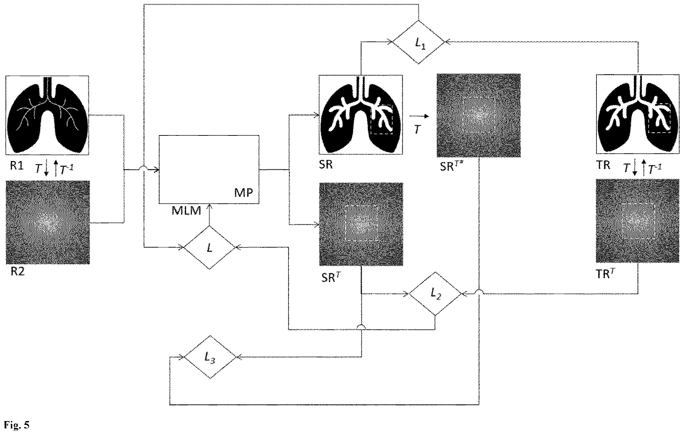

Die synthetische Repräsentation SR und die transformierte synthetische Repräsentation $SR^T$ müssen sich genauso ineinander umwandeln lassen wie die Ziel-Repräsentation TR und die transformierte Zielrepräsentation $TR^T$. Es ist möglich, eine weitere Fehlerfunktion einzuführen, die die Qualität einer solchen Umwandlung bewertet. Es ist also möglich, durch die Transformation T aus der synthetischen Repräsentation SR eine transformierte synthetische Repräsentation zu erzeugen und die Abweichungen zwischen dieser durch Transformation erzeugten und der von dem Modell des maschinellen Lernens erzeugten transformierten synthetischen Repräsentation mittels einer dritten Fehlerfunktion $L_3$ zu quantifizieren. Alternativ oder zusätzlich ist es möglich, durch die inverse Transformation $T^{-1}$ aus der transformierten synthetischen Repräsentation $SR^T$ eine synthetische Repräsentation zu erzeugen und die Abweichungen zwischen dieser durch inverse Transformation erzeugten und der von dem Modell des maschinellen Lernens erzeugten synthetischen Repräsentation mittels einer dritten Fehlerfunktion $L_3$ zu quantifizieren. Dies ist schematisch in Fig. 5 am Beispiel der Erzeugung einer transformierten synthetischen Repräsentation $SR^{T*}$ durch Transformation T aus der synthetischen Repräsentation SR gezeigt. In dem in Fig. 5 gezeigten Beispiel quantifiziert die Fehlerfunktion $L_3$ die Abweichungen zwischen der durch Transformation erzeugten transformierten synthetischen Repräsentation $SR^{T*}$ und der vom Modell des maschinellen Lernens MLM erzeugten transformierten synthetischen Repräsentation $SR^T$. Die Fehlerfunktionen $L_1$, $L_2$ und $L_3$ werden in einer Gesamtfehlerfunktion $L$ kombiniert (z.B. durch Addition). In der Gesamtfehlerfunktion $L$ können die einzelnen Terme unterschiedliche Gewichte tragen, wobei die Gewichte im Verlauf des Trainings konstant gehalten oder variiert werden können.

Eine Abwandlung von den in Fig. 4 und Fig. 5 gezeigten Ausführungsformen besteht darin, dass dem Modell des maschinellen Lernens MLM nicht beide Eingangs-Repräsentationen R1 und R2 zugeführt werden, sondern nur eine der beiden. Das Modell des maschinellen Lernens kann konfiguriert sein und trainiert werden, die jeweils andere zu erzeugen.

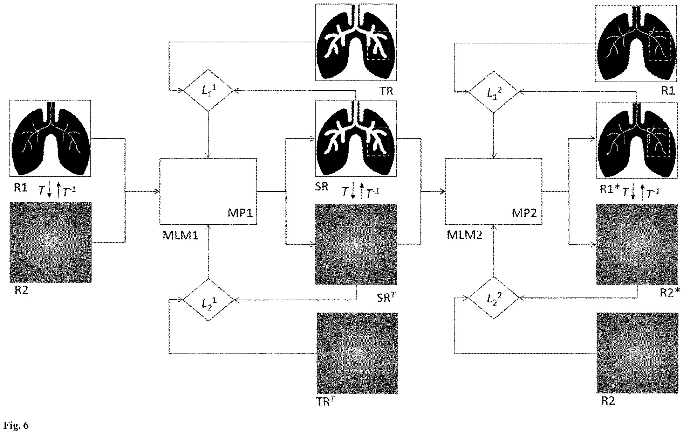

Fig. 6 zeigt schematisch eine weitere Ausführungsform des Trainingsverfahrens. In dieser Ausführungsform werden zwei Modelle des maschinellen Lernens verwendet, ein erstes Modell MLM1 und ein zweites Modell MLM2. Das erste Modell des maschinellen Lernens MLM1 wird trainiert, aus einer Eingabe-Repräsentation R1 und/oder R2 eine Ziel-Repräsentation TR und/oder eine transformierte Ziel-Repräsentation $TR^T$ vorherzusagen. Das zweite Modell des maschinellen Lernens MLM2 wird trainiert, aus einer vorhergesagten Ziel-Repräsentation SR und/oder einer vorhergesagten transformierten Ziel-Repräsentation $SR^T$ wieder die ursprüngliche Eingabe-Repräsentation R1 und/oder R2 vorherzusagen (zu rekonstruieren). Die Modelle des maschinellen Lernens führen also einen Zyklus durch, mit dessen Hilfe die Vorhersagequalität des ersten Modells MLM1 (das bei der späteren Vorhersage verwendet wird) verbessert werden kann.

Dem ersten Modell des maschinellen Lernens MLM1 wird mindestens eine Eingabe-Repräsentation R1 und/oder mindestens eine transformierte Eingabe-Repräsentation R2 als Eingabedaten zugeführt. Das erste Modell MLM1 ist konfiguriert, eine synthetische Repräsentation SR und/oder eine transformierte synthetische Repräsentation $SR^T$ auf Basis der Eingabedaten und Modellparametern MP1 zu erzeugen. Eine transformierte synthetische Repräsentation $SR^T$ kann auch durch Transformation T der synthetischen Repräsentation SR erzeugt werden. Eine synthetische Repräsentation SR kann auch durch inverse Transformation $T^{-1}$ der transformierten synthetischen Repräsentation $SR^T$ erzeugt werden.

Abweichungen zwischen der synthetischen Repräsentation SR und der Ziel-Repräsentation TR können mittels einer Fehlerfunktion $L_1^1$ quantifiziert werden. Abweichungen zwischen der transformierten synthetischen Repräsentation $SR^T$ und der transformierten Ziel-Repräsentation $TR^T$ können mittels einer Fehlerfunktion $L_2^1$ quantifiziert werden.

Abweichungen zwischen einer durch Transformation T gewonnenen synthetischen Repräsentation und der durch das Modell MLM1 erzeugten synthetischen Repräsentation SR können mittels einer Fehlerfunktion $L_3^1$ quantifiziert werden (in Fig. 6 nicht gezeigt). Alternativ oder zusätzlich können Abweichungen zwischen einer durch Transformation T gewonnenen transformierten synthetischen Repräsentation und der durch das Modell MLM1 erzeugten transformierten synthetischen Repräsentation $SR^T$ mittels einer Fehlerfunktion $L_4^1$ quantifiziert werden (in Fig. 6 nicht gezeigt).

Die Fehlerfunktionen $L_1^1$, $L_2^1$ und, falls vorhanden, $L_3^1$ und/oder $L_4^1$ können zu einer Gesamtfehlerfunktion kombiniert werden (in Fig. 6 nicht gezeigt).

Modellparameter MP1 können im Hinblick auf eine Reduzierung des Gesamtfehlers modifiziert werden.

Die synthetische Repräsentation SR und/oder die transformierte synthetische Repräsentation $SR^T$ wird/werden dem zweiten Modell des maschinellen Lernens MLM2 zugeführt. Das zweite Modell MLM2 ist konfiguriert, die erste Eingabe-Repräsentation R1 und/der die zweite Eingabe-Repräsentation R2 zu rekonstruieren (vorherzusagen). Das zweite Modell MLM2 ist konfiguriert, eine vorhergesagte erste Eingabe-Repräsentation R1* und/oder eine vorhergesagte zweite Eingabe-Repräsentation R2* auf Basis der synthetischen Repräsentation SR, der transformierten synthetischen Repräsentation $SR^T$ und auf Basis von Modellparametern MP2 zu erzeugen. Eine zweite Eingabe-Repräsentation R2* kann auch durch Transformation $T$ der ersten Eingabe-Repräsentation R1* erzeugt werden und/oder eine erste Eingabe-Repräsentation R1* kann auch durch inverse Transformation $T^{-1}$ der zweiten Eingabe-Repräsentation R2* erzeugt werden.

Abweichungen zwischen der vorhergesagten ersten Eingabe-Repräsentation R1* und der ersten Eingabe-Repräsentation R1 können mittels einer Fehlerfunktion $L_1^2$ quantifiziert werden. Abweichungen zwischen der vorhergesagten zweiten Eingabe-Repräsentation R2* und der zweiten Eingabe-Repräsentation R2 können mittels einer Fehlerfunktion $L_2^2$ quantifiziert werden.

Abweichungen zwischen einer durch inverse Transformation $T^{-1}$ gewonnenen Eingabe-Repräsentation R1* und der durch das Modell MLM2 erzeugten Eingabe-Repräsentation R1* können mittels einer Fehlerfunktion $L_3^2$ quantifiziert werden (in Fig. 6 nicht gezeigt). Alternativ oder zusätzlich können Abweichungen zwischen einer durch Transformation gewonnenen Eingabe-Repräsentation R2* und der durch das Modell MLM2 erzeugten Eingabe-Repräsentation R2* mittels einer Fehlerfunktion $L_4^2$ quantifiziert werden (in Fig. 6 nicht gezeigt).

Die Fehlerfunktionen $L_1^2$, $L_2^2$ und, falls vorhanden, $L_3^2$ und/oder $L_4^2$ können zu einer Gesamtfehlerfunktion kombiniert werden (in Fig. 6 nicht gezeigt).

Modellparameter MP2 können im Hinblick auf eine Reduzierung des Gesamtfehlers modifiziert werden.

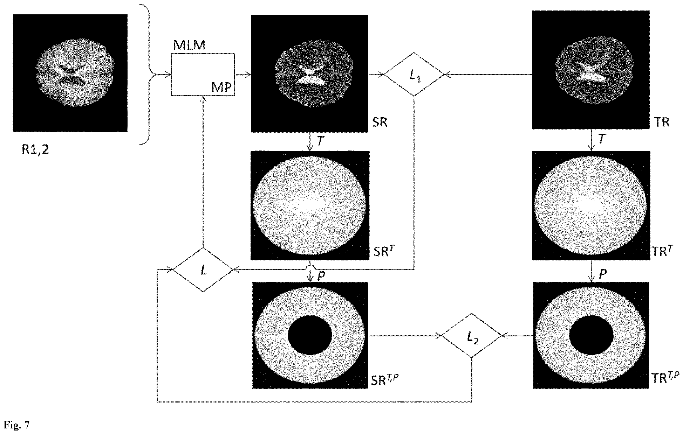

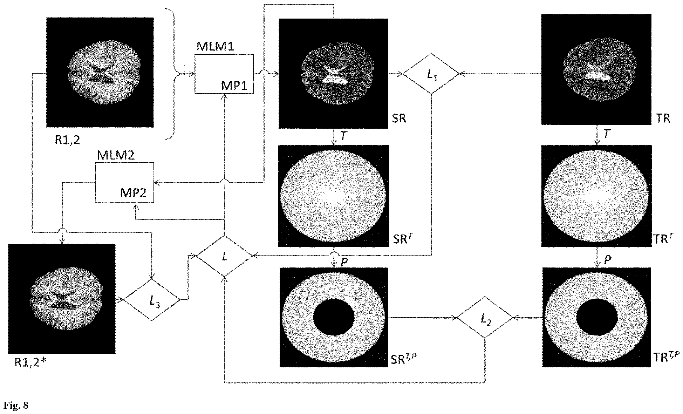

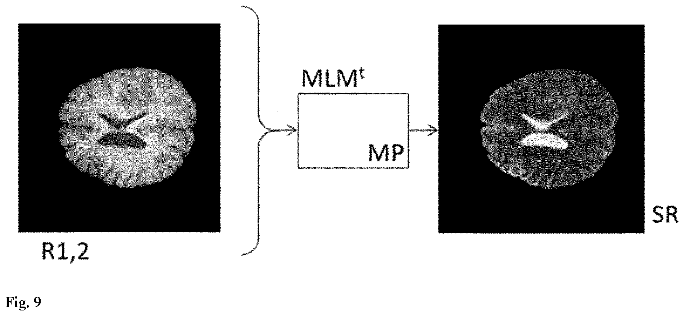

Fig. 7, Fig. 8 und Fig. 9 zeigen schematisch weitere Beispiele des Verfahrens zum Trainieren des Modells des maschinellen Lernens und zur Verwendung des trainierten Modells des maschinellen Lernens zur Vorhersage. Fig. 7 und Fig. 8 zeigen jeweils das Trainingsverfahren; Fig. 9 zeigt das Vorhersageverfahren.

In dem in Fig. 7 gezeigten Beispiel wird das Modell des maschinellen Lernens MLM trainiert, auf Basis einer oder mehrerer Eingabe-Repräsentationen R1,2, die einen Untersuchungsbereich eines Untersuchungsobjekts vor und/oder nach der Applikation einer ersten Menge eines Kontrastmittels repräsentieren, und auf Basis von Modellparametern MP eine synthetische Repräsentation SR des Untersuchungsbereichs des Untersuchungsobjekts zu erzeugen, wobei die synthetische Repräsentation SR den Untersuchungsbereich nach der Applikation einer zweiten Menge des Kontrastmittels repräsentiert, wobei die zweite Menge größer ist als die erste Menge. Mit anderen Worten: das Modell des maschinellen Lernens wird trainiert, eine Repräsentation des Untersuchungsbereichs nach der Applikation einer größeren Menge an Kontrastmittel vorherzusagen als tatsächlich appliziert worden ist. Eine Ziel-Repräsentation TR repräsentiert den Untersuchungsbereich des Untersuchungsobjekts nach der Applikation der zweiten (größeren) Menge des Kontrastmittels.

Im vorliegenden Beispiel ist der Untersuchungsbereich ein Gehirn eines Menschen. Die mindestens eine Eingabe-Repräsentation R1,2, die Ziel-Repräsentation TR und die synthetische Repräsentation SR repräsentieren das Gehirn im Ortsraum.

Die mindestens eine Eingabe-Repräsentation R1,2 wird in das Modell des maschinellen Lernens MLM eingegeben. Das Modell des maschinellen Lernens erzeugt die synthetische Repräsentation SR.

Mittels einer Transformation $T$ wird die synthetische Repräsentation SR in eine transformierte synthetische Transformation $SR^T$ transformiert. Die transformierte synthetische Transformation $SR^T$ wird mit Hilfe einer Funktion $P$ auf einen Teil reduziert; es entsteht eine anteilige transformierte synthetische Transformation $SR^{T,P}$.

Analog werden aus der Ziel-Repräsentation SR mittels der Transformation $T$ eine transformierte Ziel-Repräsentation $TR^T$ und mittels der Funktion $P$ aus der transformierten Ziel-Repräsentation $TR^T$ eine anteilige transformierte Ziel-Repräsentation $TR^{T,P}$ erzeugt.

Die transformierte synthetische Repräsentation $SR^T$ und die transformierte Ziel-Repräsentation $TR^T$ repräsentieren den Untersuchungsbereich im Frequenzraum. Die anteilige transformierte synthetische Repräsentation $SR^{T,P}$ ist eine auf einen Frequenzbereich mit höheren Frequenzen reduzierte transformierte synthetische Repräsentation $SR^T$. Die anteilige transformierte Ziel-Repräsentation $TR^{T,P}$ ist eine auf den Frequenzbereich mit höheren Frequenzen reduzierte transformierte Ziel-Repräsentation $TR^T$. Das Modell des maschinellen Lernens MLM wird also trainiert, einen Fokus auf Feinstrukturinformationen zu legen, die in den höheren Frequenzen kodiert sind.

Das Modell des maschinellen Lernens wird mit Hilfe einer Fehlerfunktion $L$ trainiert. Die Fehlerfunktion $L$ quantifiziert die Abweichungen i) zwischen der synthetischen Repräsentation SR und der Ziel-Repräsentation TR mittels eines ersten Terms $L_1$ und ii) zwischen der anteiligen transformierten synthetischen Repräsentation $SR^{T,P}$ und der anteiligen transformierten Ziel-Repräsentation $TR^{T,P}$ mittels eines zweiten Terms $L_2$. Der erste Term $L_1$ und der zweite Term $L_2$ können in der Fehlerfunktion beispielsweise jeweils mit einem Gewichtsfaktor multipliziert und die resultierenden Produkte addiert werden (wie mit der Gleichung Gl. 1 beschrieben).

In einem Optimierungsverfahren, z.B. einem Gradientenverfahren, können die Modellparameter MP im Hinblick auf eine Reduzierung des mittels der Funktion $L$ berechneten Fehlers modifiziert werden.

Der beschriebene Prozess wird für weitere Untersuchungsobjekte wiederholt. Das Training kann beendet werden, wenn die mittels der Fehlerfunktion $L$ errechneten Fehler ein definiertes Minimum erreichen, d.h. die Vorhersagequalität ein gewünschtes Niveau erreicht.

Fig. 8 zeigt das in Bezug zu Fig. 7 diskutierte Trainingsverfahren mit folgenden Erweiterungen/Änderungen:

- In dem Trainingsverfahren werden zwei Modelle des maschinellen Lernens eingesetzt, ein erstes Modell MLM1 und ein zweites Modell MLM2.
- Das erste Modell des maschinellen Lernens MLM1 führt die Funktionen aus, die für das Modell des maschinellen Lernens MLM in Bezug zu Fig. 7 beschrieben wurden.
- Das zweite Modell des maschinellen Lernens ist konfiguriert und wird trainiert, die mindestens eine Eingabe-Repräsentation R1,2 zu reproduzieren. Mit anderen Worten, das zweite Modell des maschinellen Lernens ist konfiguriert und wird trainiert, auf Basis der synthetischen Repräsentation SR und auf Basis von Modellparametern MP2 eine vorhergesagte Eingabe-Repräsentation R1,2 zu erzeugen.
- Die Fehlerfunktion $L$ verfügt über einen dritten Term $L_3$, der die Abweichungen zwischen der Eingabe-Repräsentation R1,2 und der vorhergesagten Eingabe-Repräsentation R1,2* quantifiziert.
- In einem Optimierungsverfahren, z.B. einem Gradientenverfahren, können die Modellparameter MP1 und MP2 im Hinblick auf eine Reduzierung des mittels der Funktion $L$ berechneten Fehlers modifiziert werden.

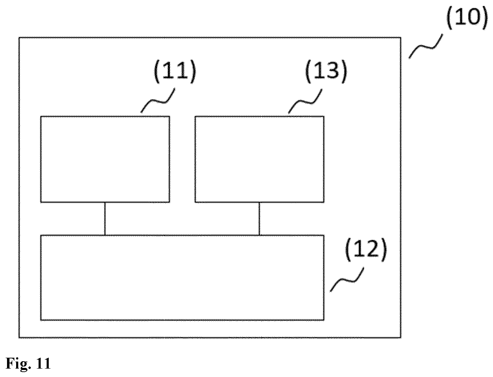

Fig. 9 zeigt die Verwendung des gemäß Fig. 7 oder Fig. 8 trainierten Modell des maschinellen Lernens MLM oder MLM1, in Fig. 11 mit MLM$^t$ gekennzeichnet.

Dem trainierten Modell des maschinellen Lernens MLM$^t$ wird mindestens eine Eingabe-Repräsentation R1,2, die das Gehirn eines neuen Untersuchungsobjekts repräsentiert, zugeführt. Der Begriff "neu" bedeutet, dass die mindestens eine Eingabe-Repräsentation R1,2 nicht bereits im Training verwendet wurde. Die Eingabe-Repräsentation R1,2 repräsentiert das Gehirn des neuen Untersuchungsobjekts vor und/oder nach der Applikation einer ersten Menge eines Kontrastmittels.

Das trainierte Modell des maschinellen Lernens erzeugt auf Basis der mindestens einen Eingabe-Repräsentation R1,2 eine synthetische Repräsentation SR, die das Gehirn nach der Applikation einer zweiten Menge des Kontrastmittels repräsentiert.

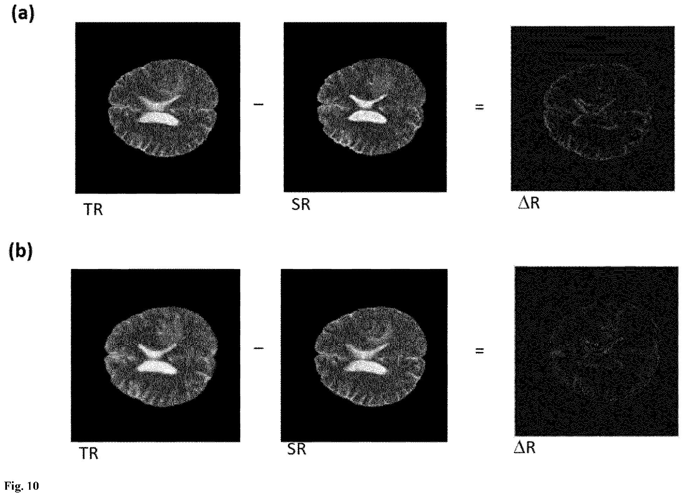

Fig. 10 zeigt das Ergebnis einer Validierung (a) des gemäß Fig. 7 trainierten Modells des maschinellen Lernens und (b) des gemäß Fig. 8 trainierten Modells des maschinellen Lernens. Jedem der Modelle wurde wie in Bezug auf Fig. 9 beschrieben mindestens eine Eingabe-Repräsentation eines neuen Untersuchungsobjekts zugeführt. Jedes der Modelle erzeugt jeweils eine synthetische Repräsentation SR. Für das Untersuchungsobjekt liegt eine Ziel-Repräsentation TR vor. In Fig. 10 ist das Erzeugen einer Differenz-Repräsentation gezeigt, wobei für jedes Modell eine Differenz-Repräsentation ΔR durch Subtraktion der jeweiligen synthetischen Repräsentation SR von der Ziel-Repräsentation TR erzeugt wird. Im Falle eines perfekten Modells entspricht jeder der synthetischen Repräsentationen SR der ZielRepräsentation TR und die Differenz-Repräsentation ΔR ist komplett schwarz (alle Werte sind Null). In Fig. 10 ist zu erkennen, dass im Fall des gemäß Fig. 7 trainierten Modells des maschinellen Lernens (Fig. 10 (a)) mehr Pixel von Null verschieden sind als im Fall des gemäß Fig. 8 trainierten Modell des maschinellen Lernens (Fig. 10 (b)). Die Vorhersagequalität von dem gemäß Fig. 8 trainierten Modell des maschinellen Lernens ist im vorliegenden Beispiel also höher als die von dem gemäß Fig. 7 trainierten Modell des maschinellen Lernens.

Bei den Modellen des maschinellen Lernens gemäß der vorliegenden Offenbarung kann es sich beispielsweise um ein künstliches neuronales Netzwerk handeln, oder es kann ein oder mehrere solcher künstlichen neuronalen Netze umfassen.

Ein künstliches neuronales Netzwerk umfasst mindestens drei Schichten von Verarbeitungselementen: eine erste Schicht mit Eingangsneuronen (Knoten), eine N-te Schicht mit mindestens einem Ausgangsneuron (Knoten) und N-2 innere Schichten, wobei N eine natürliche Zahl und größer als 2 ist.

Die Eingangsneuronen dienen zum Empfangen der Eingabe-Repräsentationen. Üblicherweise gibt es ein Eingangsneuron für jedes Pixel oder Voxel einer Eingabe-Repräsentation, falls es sich bei der Repräsentation um eine Ortsraumdarstellung in Form einer Rastergrafik handelt, oder ein Eingangsneuron für jede Frequenz, die in der Eingabe-Repräsentation vorhanden ist, falls es sich bei der Repräsentation um eine Frequenzraumdarstellung handelt. Es können zusätzliche Eingangsneuronen für zusätzliche Eingangswerte (z.B. Informationen zum Untersuchungsbereich, zum Untersuchungsobjekt, zu Bedingungen, die bei der Erzeugung der Eingabe-Repräsentation herrschten, Informationen zu dem Zustand, den die Eingabe-Repräsentation repräsentiert, und/oder Informationen zu dem Zeitpunkt oder der Zeitspanne zu/in der die Eingabe-Repräsentation erzeugt worden ist) vorhanden sein.

Die Ausgangsneuronen können dazu dienen, eine synthetische Repräsentation, die den Untersuchungsbereich in einem anderen Zustand repräsentiert, auszugeben.

Die Verarbeitungselemente der Schichten zwischen den Eingangsneuronen und den Ausgangsneuronen sind in einem vorbestimmten Muster mit vorbestimmten Verbindungsgewichten miteinander verbunden.

Vorzugsweise handelt es sich bei dem künstlichen neuronalen Netz um ein so genanntes Convolutional Neural Network (kurz: CNN) oder es umfasst ein solches.

Ein CNN besteht üblicherweise im Wesentlichen aus Filtern (Convolutional Layer) und Aggregations-Schichten (Pooling Layer), die sich abwechselnd wiederholen, und am Ende aus einer Schicht oder mehreren Schichten von "normalen" vollständig verbundenen Neuronen (Dense / Fully Connected Layer).

Das Trainieren des neuronalen Netzes kann beispielsweise mittels eines Backpropagation-Verfahrens durchgeführt werden. Dabei wird für das Netz eine möglichst zuverlässige Abbildung der Eingabe-Repräsentation(en) auf die synthetische Repräsentation angestrebt. Die Qualität der Vorhersage wird durch eine Fehlerfunktion beschrieben. Das Ziel ist die Minimierung der Fehlerfunktion. Das Einlernen eines künstlichen neuronalen Netzes erfolgt bei dem Backpropagation-Verfahren durch die Änderung der Verbindungsgewichte.

Im trainierten Zustand enthalten die Verbindungsgewichte zwischen den Verarbeitungselementen Informationen bezüglich der Dynamik der Beziehung zwischen der/den Eingabe-Repräsentation(en) und der synthetischen Repräsentation, die verwendet werden können, um, auf Basis einer oder mehrerer Repräsentationen des Untersuchungsbereichs eines neuen Untersuchungsobjekts eine synthetische Repräsentation des Untersuchungsbereichs des neuen Untersuchungsobjekts vorherzusagen. Dabei bedeutet der Begriff "neu", dass Repräsentationen des Untersuchungsbereichs des neuen Untersuchungsobjekt nicht bereits beim Trainieren des Modells des maschinellen Lernens verwendet wurden.

Eine Kreuzvalidierungsmethode kann verwendet werden, um die Daten in Trainings- und Validierungsdatensätze aufzuteilen. Der Trainingsdatensatz wird beim Backpropagation-Training der Netzwerkgewichte verwendet. Der Validierungsdatensatz wird verwendet, um zu überprüfen, mit welcher Vorhersagegenauigkeit sich das trainierte Netzwerk auf unbekannte (neue) Daten anwenden lässt.

Das künstliche neuronale Netzwerk kann eine Autoencoder-Architektur aufweisen; z.B. kann das künstliche neuronale Netzwerk eine Architektur wie das *U-Net* aufweisen (siehe z.B. O. Ronneberger et al.: U-net: Convolutional networks for biomedical image segmentation, International Conference on Medical image computing and computer-assisted intervention, Seiten 234-241, Springer, 2015, https://doi.org/10.1007/978-3-319-24574-4_28).

Das künstliche neuronale Netzwerk kann ein *Generative Adversarial Network* (GAN) sein (siehe z.B. M.-Y. Liu et al.: Generative Adversarial Networks for Image and Video Synthesis: Algorithms and Applications, arXiv:2008.02793; J. Henry et al.: Pix2Pix GAN for Image-to-Image Translation, DOI: 10.13140/RG.2.2.32286.66887).

Das künstliche neuronale Netz kann ein *Regularized Generative Adversarial Network* sein (siehe z.B. Q. Li et al.: RegGAN: An End-to-End Network for Building Footprint Generation with Boundary Regularization, Remote Sens. 2022, 14, 1835).

Das künstliche neuronale Netzwerk kann ein *Conditional Adversarial Network* sein (siehe z.B. P. Isola et al.: Image-to-Image Translation with Conditional Adversarial Networks, arXiv:1611.07004 [cs.CV]).

Das künstliche neuronale Netz kann ein Transformer-Netzwerk sein (siehe z.B. D. Karimi et al.: Convolution-Free Medical Image Segmentation using Transformers, arXiv:2102.13645 [eess.IV]).

Fig. 11 zeigt beispielhaft und schematisch ein Computersystem gemäß der vorliegenden Offenbarung und das zum Trainieren des Modells des maschinellen Lernens und/oder zur Verwendung des trainierten Modells des maschinellen Lernens zur Vorhersage verwendet werden kann.

Ein "Computersystem" ist ein System zur elektronischen Datenverarbeitung, das mittels programmierbarer Rechenvorschriften Daten verarbeitet. Ein solches System umfasst üblicherweise einen "Computer", diejenige Einheit, die einen Prozessor zur Durchführung logischer Operationen umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Laufwerke, Kamera, Mikrofon, Lautsprecher etc. Auch interne Anschlüsse und Erweiterungskarten gelten in der Computertechnik als Peripherie.

Das in Fig. 11 gezeigte Computersystem (1) umfasst eine Eingabeeinheit (10), eine Steuer- und Recheneinheit (20) und eine Ausgabeeinheit (30).

Die Steuer- und Recheneinheit (20) dient der Steuerung des Computersystems (1), der Koordinierung der Datenflüsse zwischen den Einheiten des Computersystems (1) und der Durchführung von Berechnungen.

Die Steuer- und Recheneinheit (20) ist konfiguriert:

- die Empfangseinheit (10) zu veranlassen, mindestens eine Eingabe-Repräsentation eines Untersuchungsbereichs eines neuen Untersuchungsobjekts zu empfangen, wobei die mindestens eine Eingabe-Repräsentation den Untersuchungsbereich vor und/oder nach der Applikation einer ersten Menge eines Kontrastmittels repräsentiert,

- die empfangene mindestens eine Eingabe-Repräsentation in ein trainiertes Modell des maschinellen Lernens einzugeben, wobei das trainierte Modell des maschinellen Lernens wie in dieser Beschreibung beschrieben trainiert worden ist,

- von dem Modell des maschinellen Lernens eine synthetische Repräsentation des Untersuchungsbereichs des neuen Untersuchungsobjekts zu empfangen, wobei die synthetische Repräsentation den Untersuchungsbereich nach der Applikation einer zweiten Menge des Kontrastmittels repräsentiert, wobei die zweite Menge verschieden von der ersten Menge ist,

- die Ausgabeeinheit (30) zu veranlassen, die synthetische Repräsentation auszugeben und/oder zu speichern und/oder an ein separates Computersystem zu übermitteln.

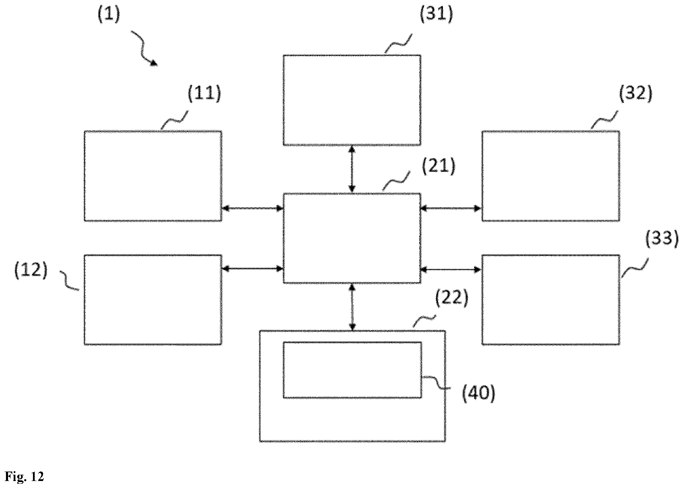

Fig. 12 zeigt beispielhaft und schematisch eine weitere Ausführungsform des erfindungsgemäßen Computersystems.

Das Computersystem (1) umfasst eine Verarbeitungseinheit (21), die mit einem Speicher (22) verbunden ist. Die Verarbeitungseinheit (21) und der Speicher (22) bilden eine Steuer- und Berechnungseinheit, wie sie in Fig. 11 gezeigt ist.

Die Verarbeitungseinheit (21) (engl.: *processing unit)* kann einen oder mehrere Prozessoren allein oder in Kombination mit einem oder mehreren Speichern umfassen. Bei der Verarbeitungseinheit (21) kann es sich um gewöhnliche Computerhardware handeln, die in der Lage ist, Informationen wie z.B. digitale Bildaufnahmen, Computerprogramme und/oder andere digitale Informationen zu verarbeiten. Die Verarbeitungseinheit (21) besteht üblicherweise aus einer Anordnung elektronischer Schaltungen, von denen einige als integrierter Schaltkreis oder als mehrere miteinander verbundene integrierte Schaltkreise (ein integrierter Schaltkreis wird manchmal auch als "Chip" bezeichnet) ausgeführt sein können. Die Verarbeitungseinheit (21) kann konfiguriert sein, Computerprogramme auszuführen, die in einem Arbeitsspeicher der Verarbeitungseinheit (21) oder im Speicher (22) desselben oder eines anderen Computersystems gespeichert sein können.

Der Speicher (22) kann eine gewöhnliche Computerhardware sein, die in der Lage ist, Informationen wie z.B. digitale Bildaufnahmen (z.B. Repräsentationen des Untersuchungsbereichs), Daten, Computerprogramme und/oder andere digitale Informationen entweder vorübergehend und/oder dauerhaft zu speichern. Der Speicher (22) kann einen flüchtigen und/oder nichtflüchtigen Speicher umfassen und kann fest eingebaut oder entfernbar sein. Beispiele für geeignete Speicher sind RAM (Random Access Memory), ROM (Read-Only Memory), eine Festplatte, ein Flash-Speicher, eine austauschbare Computerdiskette, eine optische Disc, ein Magnetband oder eine Kombination der oben genannten. Zu den optischen Discs können Compact Discs mit Nur-Lese-Speicher (CD-ROM), Compact Discs mit Lese-/Schreibfunktion (CD-R/W), DVDs, Blu-ray-Discs und ähnliche gehören.

Zusätzlich zum Speicher (22) kann die Verarbeitungseinheit (21) auch mit einer oder mehreren Schnittstellen (11, 12, 31, 32, 33) verbunden sein, um Informationen anzuzeigen, zu übertragen und/oder zu empfangen. Die Schnittstellen können eine oder mehrere Kommunikationsschnittstellen (32, 33) und/oder eine oder mehrere Benutzerschnittstellen (11, 12, 31) umfassen. Die eine oder mehrere Kommunikationsschnittstellen können so konfiguriert sein, dass sie Informationen senden und/oder empfangen, z.B. zu und/oder von einer MRT-Scanner, einem CT-Scanner, einer Ultraschallkamera, anderen Computersystemen, Netzwerken, Datenspeichern oder dergleichen. Die eine oder mehrere Kommunikationsschnittstellen können so konfiguriert sein, dass sie Informationen über physische (verdrahtete) und/oder drahtlose Kommunikationsverbindungen übertragen und/oder empfangen. Die eine oder die mehreren Kommunikationsschnittstellen können eine oder mehrere Schnittstellen für die Verbindung mit einem Netzwerk enthalten, z.B. unter Verwendung von Technologien wie Mobiltelefon, Wi-Fi, Satellit, Kabel, DSL, Glasfaser und/oder dergleichen. In einigen Beispielen können die eine oder die mehreren Kommunikationsschnittstellen eine oder mehrere Nahbereichskommunikationsschnittstellen umfassen, die so konfiguriert sind, dass sie Geräte mit Nahbereichskommunikationstechnologien wie NFC, RFID, Bluetooth, Bluetooth LE, ZigBee, Infrarot (z. B. IrDA) oder Ähnlichem verbinden.

Die Benutzerschnittstellen können eine Anzeige (31) umfassen. Eine Anzeige (31) kann so konfiguriert sein, dass sie einem Benutzer Informationen anzeigt. Geeignete Beispiele hierfür sind eine Flüssigkristallanzeige (LCD), eine Leuchtdiodenanzeige (LED), ein Plasmabildschirm (PDP) oder Ähnliches. Die Benutzereingabeschnittstelle(n) (11, 12) kann/können verdrahtet oder drahtlos sein und kann/können so konfiguriert sein, dass sie Informationen von einem Benutzer in das Computersystem (1) empfängt/empfangen, z.B. zur Verarbeitung, Speicherung und/oder Anzeige. Geeignete Beispiele für Benutzereingabeschnittstellen sind ein Mikrofon, ein Bild- oder Videoaufnahmegerät (z.B. eine Kamera), eine Tastatur oder ein Tastenfeld, ein Joystick, eine berührungsempfindliche Oberfläche (getrennt von einem Touchscreen oder darin integriert) oder ähnliches. In einigen Beispielen können die Benutzerschnittstellen eine automatische Identifikations- und Datenerfassungstechnologie (AIDC) für maschinenlesbare Informationen enthalten. Dazu können Barcodes, Radiofrequenz-Identifikation (RFID), Magnetstreifen, optische Zeichenerkennung (OCR), Karten mit integrierten Schaltkreisen (ICC) und ähnliches gehören. Die Benutzerschnittstellen können ferner eine oder mehrere Schnittstellen für die Kommunikation mit Peripheriegeräten wie Druckern und dergleichen umfassen.

Ein oder mehrere Computerprogramme (40) können im Speicher (22) gespeichert sein und von der Verarbeitungseinheit (21) ausgeführt werden, die dadurch programmiert wird, die in dieser Beschreibung beschriebenen Funktionen zu erfüllen. Das Abrufen, Laden und Ausführen von Anweisungen des Computerprogramms (40) kann sequenziell erfolgen, so dass jeweils ein Befehl abgerufen, geladen und ausgeführt wird. Das Abrufen, Laden und/oder Ausführen kann aber auch parallel erfolgen.

In dem Speicher (22) kann auch das erfindungsgemäße Modell des maschinellen Lernens gespeichert sein.

Das erfindungsgemäße System kann als Lap-

top, Notebook, Netbook und/ der Tablet-PC ausgeführt sein, es kann auch ein Bestandteil eines MRT-Scanners, eines CT-Scanners oder eines Ultraschalldiagnosegeräts sein.

**Patentansprüche**

1. Verfahren zum Trainieren eines Modells (MLM) des maschinellen Lernens zur Erzeugung synthetischer radiologischer Aufnahmen auf Basis von gemessenen radiologischen Aufnahmen, wobei das Verfahren umfasst:

- Empfangen und/oder Bereitstellen von Trainingsdaten (TD), wobei die Trainingsdaten für jedes Untersuchungsobjekt einer Vielzahl von Untersuchungsobjekten einen Satz an Eingabedaten und Zieldaten umfasst,

wobei jeder Satz

∘ mindestens eine Eingabe-Repräsentation (R1, R2) eines Untersuchungsbereichs des Untersuchungsobjekts in einem ersten Zustand als Eingabedaten und
∘ eine Ziel-Repräsentation (TR) des Untersuchungsbereichs des Untersuchungsobjekts in einem zweiten Zustand sowie eine transformierte Ziel-Repräsentation ($TR^T$) als Zieldaten umfasst,

wobei die transformierte Ziel-Repräsentation ($TR^T$) zumindest einen Teil des Untersuchungsbereichs des Untersuchungsobjekts

∘ im Frequenzraum repräsentiert, falls die Ziel-Repräsentation (TR) den Untersuchungsbereichs des Untersuchungsobjekts im Ortsraum repräsentiert, oder
∘ im Ortsraum repräsentiert, falls die Ziel-Repräsentation (TR) den Untersuchungsbereich des Untersuchungsobjekts im Frequenzraum repräsentiert,

- Trainieren eines Modells (MLM) des maschinellen Lernens, wobei das Modell (MLM) des maschinellen Lernens konfiguriert ist, auf Basis mindestens einer Eingabe-Repräsentation (R1, R2) und Modellparametern (MP) eine synthetische Repräsentation (SR) des Untersuchungsbereichs des Untersuchungsobjekts zu erzeugen, wobei die mindestens eine Eingabe-Repräsentation (R1, R2) den Untersuchungsbereich vor und/oder nach der Applikation einer ersten Menge eines Kontrastmittels repräsentiert, und die synthetische Repräsentation (SR) den Untersuchungsbereich nach der Applikation einer zweiten Menge des Kontrastmittels repräsentiert, wobei die erste Menge verschieden von der zweiten Menge ist,

wobei das Trainieren für jedes Untersuchungsobjekt der Vielzahl an Untersuchungsobjekten umfasst:

∘ Zuführen der mindestens einen Eingabe-Repräsentation (R1, R2) dem Modell (MLM) des maschinellen Lernens,
∘ Empfangen einer synthetischen Repräsentation (SR) des Untersuchungsbereichs des Untersuchungsobjekts von dem Modell (MLM) des maschinellen Lernens,
∘ Erzeugen und/oder Empfangen einer transformierten synthetischen Repräsentation ($SR^T$) auf Basis und/oder zu der synthetischen Repräsentation (SR), wobei die transformierte synthetische Repräsentation ($SR^T$) zumindest einen Teil des Untersuchungsbereichs des Untersuchungsobjekts

■ im Frequenzraum repräsentiert, falls die synthetische Repräsentation (SR) den Untersuchungsbereichs des Untersuchungsobjekts im Ortsraum repräsentiert, oder
■ im Ortsraum repräsentiert, falls die synthetische Repräsentation (SR) den Untersuchungsbereich des Untersuchungsobjekts im Frequenzraum repräsentiert,

∘ Quantifizieren der Abweichungen i) zwischen zumindest einem Teil der synthetischen Repräsentation (SR) und zumindest einem Teil der Ziel-Repräsentation (TR) und ii) zwischen zumindest einem Teil ($SR^{T,P}$) der transformierten synthetischen Repräsentation ($SR^T$) und zumindest einem Teil ($TR^{T,P}$) der transformierten Ziel-Repräsentation ($TR^T$) mittels einer Fehlerfunktion (L),
∘ Modifizieren von Modellparametern (MP) im Hinblick auf reduzierte Abweichungen,

- Ausgeben und/oder Speichern des trainierten Modells ($MLM^t$) des maschinellen Lernens und/oder der Modellparameter (MP) und/oder Übermitteln des trainierten Modells ($MLM^t$) des maschinellen Lernens und/oder der Modellparameter (MP) an ein separates Computersystem.

2. Verfahren gemäß Anspruch 1,

wobei das Empfangen und/oder Bereitstellen von Trainingsdaten (TD) umfasst:

- Erzeugen einer anteiligen transformierten Ziel-Repräsentation ($TR^{T,P}$), wobei die anteilige transformierte Ziel-Repräsentation ($TR^{T,P}$) auf einen Teil oder mehrere Teile der transformierten Ziel-Repräsentation ($TR^T$) reduziert wird,

wobei das Erzeugen und/oder Empfangen einer transformierten synthetischen Repräsentation ($SR^T$) auf Basis und/oder zu der synthetischen Repräsentation (SR) umfasst:

- Erzeugen einer anteiligen transformierten synthetischen Repräsentation ($SR^{T,P}$), wobei die anteilige transformierte synthetische Repräsentation ($SR^{T,P}$) auf einen Teil oder mehrere Teile der transformierten synthetischen Repräsentation ($SR^T$) reduziert wird,

wobei das Quantifizieren der Abweichungen zwischen der transformierten synthetischen Repräsentation ($SR^T$) und der transformierten Ziel-Repräsentation ($TR^T$) umfasst:

- Quantifizieren der Abweichungen zwischen der anteiligen transformierten synthetischen ($SR^{T,P}$) Repräsentation und der anteiligen transformierten Ziel-Repräsentation ($TR^{T,P}$).

3. Verfahren gemäß Anspruch 1 oder 2,
wobei das Trainieren für jedes Untersuchungsobjekt der Vielzahl an Untersuchungsobjekten umfasst:

○ Zuführen der mindestens einen Eingabe-Repräsentation (R1, R2) dem Modell (MLM) des maschinellen Lernens,
○ Empfangen der synthetischen Repräsentation (SR) und einer ersten transformierten synthetischen Repräsentation ($SR^T$) des Untersuchungsbereichs des Untersuchungsobjekts von dem Modell (MLM) des maschinellen Lernens, wobei die erste transformierte synthetische Repräsentation ($SR^T$) zumindest einen Teil des Untersuchungsbereichs des Untersuchungsobjekts

■ im Frequenzraum repräsentiert, falls die synthetische Repräsentation (SR) den Untersuchungsbereichs des Untersuchungsobjekts im Ortsraum repräsentiert, oder
■ im Ortsraum repräsentiert, falls die synthetische Repräsentation (SR) den Untersuchungsbereich des Untersuchungsobjekts im Frequenzraum repräsentiert,

○ Erzeugen einer zweiten transformierten synthetischen Repräsentation ($SR^{T*}$) auf Basis der synthetischen Repräsentation (SR) mittels einer Transformation (T), wobei die zweite transformierte synthetische Repräsentation ($SR^{T*}$) zumindest einen Teil des Untersuchungsbereichs des Untersuchungsobjekts

■ im Frequenzraum repräsentiert, falls die synthetische Repräsentation (SR) den Untersuchungsbereichs des Untersuchungsobjekts im Ortsraum repräsentiert, oder
■ im Ortsraum repräsentiert, falls die synthetische Repräsentation (SR) den Untersuchungsbereich des Untersuchungsobjekts im Frequenzraum repräsentiert,

- Quantifizieren der Abweichungen i) zwischen zumindest einem Teil der synthetischen Repräsentation (SR) und zumindest einem Teil der Ziel-Repräsentation (TR), ii) zwischen zumindest einem Teil der ersten transformierten synthetischen Repräsentation ($SR^T$) und zumindest einem Teil der transformierten Ziel-Repräsentation (TR*) und iii) zwischen zumindest einem Teil der ersten transformierten synthetischen Repräsentation ($SR^T$) und zumindest einem Teil der zweiten transformierten synthetischen Repräsentation ($SR^{T*}$) mittels einer Fehlerfunktion (L),
- Modifizieren von Modellparametern (MP) im Hinblick auf reduzierte Abweichungen.

4. Verfahren nach einem der Ansprüche 1 bis 3,

wobei das Modell (MLM) des maschinellen Lernens ein erstes Modell (MLM1) des maschinellen Lernens und ein zweites Modell (MLM2) des maschinellen Lernens umfasst,
wobei das erste Modell (MLM1) des maschinellen Lernens konfiguriert ist, auf Basis mindestens einer Eingabe-Repräsentation (R1) und Modellparametern (MP1) eine synthetische Repräsentation (SR) des Untersuchungsbereichs des Untersuchungsobjekts zu erzeugen,
wobei das zweite Modell (MLM2) des maschinellen Lernens konfiguriert ist, auf Basis der synthetischen Repräsentation (SR) des Untersuchungsbereichs des Untersuchungsobjekts die Eingabe-Repräsentation (R1) zu rekonstruieren,
wobei das Trainieren für jedes Untersuchungsobjekt der Vielzahl an Untersuchungsobjekten umfasst:

o Erzeugen einer transformierten Eingabe-Repräsentation (R2) auf Basis der Eingabe-Repräsentation (R1) mittels einer Transformation (T), wobei die transformierte Eingabe-Repräsentation (R2) zumindest einen Teil des Untersuchungsbereichs des Untersuchungsobjekts

■ im Frequenzraum repräsentiert, falls die Eingabe-Repräsentation (R1) den Untersuchungsbereichs des Untersuchungsobjekts im Ortsraum repräsentiert, oder
■ im Ortsraum repräsentiert, falls die Eingabe-Repräsentation (R1) den Untersuchungsbereich des Untersuchungsobjekts im Frequenzraum repräsentiert,

o Zuführen der mindestens einen Eingabe-Repräsentation (R1) dem ersten Modell (MLM1) des maschinellen Lernens,
o Empfangen einer synthetischen Repräsentation (SR) des Untersuchungsbereichs des Untersuchungsobjekts von dem ersten Modell (MLM1) des maschinellen Lernens,
o Erzeugen und/oder Empfangen einer transformierten synthetischen Repräsentation (SR$^T$) auf Basis und/oder zu der synthetischen Repräsentation (SR), wobei die transformierte synthetische Repräsentation (SR$^T$) zumindest einen Teil des Untersuchungsbereichs des Untersuchungsobjekts

■ im Frequenzraum repräsentiert, falls die synthetische Repräsentation (SR) den Untersuchungsbereichs des Untersuchungsobjekts im Ortsraum repräsentiert, oder
■ im Ortsraum repräsentiert, falls die synthetische Repräsentation (SR) den Untersuchungsbereich des Untersuchungsobjekts im Frequenzraum repräsentiert,

o Zuführen der synthetischen Repräsentation (SR) und/oder der transformierten synthetischen Repräsentation (SR$^T$) dem zweiten Modell (MLM2) des maschinellen Lernens,
∘ Empfangen einer vorhergesagten Eingabe-Repräsentation (R1*) von dem zweiten Modell (MLM2) des maschinellen Lernens,
∘ Erzeugen und/oder Empfangen einer transformierten vorhergesagten Eingabe-Repräsentation (R2*) auf Basis und/oder zu der vorhergesagten Eingabe-Repräsentation (R1*), wobei die transformierte vorhergesagte Eingabe-Repräsentation (R2*) zumindest einen Teil des Untersuchungsbereichs des Untersuchungsobjekts

■ im Frequenzraum repräsentiert, falls die vorhergesagte Eingabe-Repräsentation (R1*) den Untersuchungsbereichs des Untersuchungsobjekts im Ortsraum repräsentiert, oder
■ im Ortsraum repräsentiert, falls die vorhergesagte Eingabe-Repräsentation (R1*) den Untersuchungsbereich des Untersuchungsobjekts im Frequenzraum repräsentiert,

o Quantifizieren der Abweichungen i) zwischen zumindest einem Teil der synthetischen Repräsentation (SR) und zumindest einem Teil der Ziel-Repräsentation (TR), ii) zwischen zumindest einem Teil der transformierten synthetischen Repräsentation (SR$^T$) und zumindest einem Teil der transformierten Ziel-Repräsentation (TR$^T$), iii) zwischen zumindest einem Teil der Eingabe-Repräsentation (R1) und zumindest einem Teil der vorhergesagten Eingabe-Repräsentation (R1*) und iv) zwischen zumindest einem Teil der transformierten Eingabe-Repräsentation (R2) und zumindest einem Teil der transformierten vorhergesagten Eingabe-Repräsentation (R2*) mittels einer Fehlerfunktion,
o Modifizieren von Modellparametern (MP1, MP2) im Hinblick auf reduzierte Abweichungen.

5.　Verfahren gemäß einem der Ansprüche 1 bis 4, wobei die erste Menge geringer als die zweite Menge ist.

6.　Verfahren gemäß einem der Ansprüche 1 bis 5, wobei der Untersuchungsbereich eine Leber oder ein Teil einer Leber eines Menschen ist.

7.　Verfahren gemäß einem der Ansprüche 1 bis 6, wobei jede der mindestens einen Eingabe-Repräsentation (R1, R2) eine Repräsentation des Untersuchungsbereichs im Ortsraum ist, die ZielRepräsentation (TR) eine Repräsentation des Untersuchungsbereichs im Ortsraum ist und die synthetische Repräsentation (SR) eine Repräsentation des Untersuchungsbereichs im Ortsraum ist.

8.　Verfahren gemäß einem der Ansprüche 2 bis 6, wobei die anteilige transformierte synthetische Repräsentation (SR$^{T,P}$) den Untersuchungsbereich im Frequenzraum repräsentiert, wobei die anteilige transformierte synthetische Repräsentation ($SR^{T,P}$) auf einen Frequenzbereich der transformierten synthetischen Repräsentation ($SR^T$) reduziert wird, wobei in dem Frequenzbereich Kontrastinformationen kodiert sind.

9. Verfahren gemäß einem der Ansprüche 2 bis 6, wobei die anteilige transformierte synthetische Repräsentation ($SR^{T,P}$) den Untersuchungsbereich im Frequenzraum repräsentiert, wobei die anteilige transformierte synthetische Repräsentation ($SR^{T,P}$) auf einen Frequenzbereich der transformierten synthetischen Repräsentation ($SR^T$) reduziert wird, wobei in dem Frequenzbereich Informationen über Feinstrukturen kodiert sind.

10. Computer-implementiertes Verfahren zum Erzeugen einer synthetischen Repräsentation eines Untersuchungsbereichs eines Untersuchungsobjekts, wobei das Verfahren umfasst:

   - Empfangen mindestens einer Eingabe-Repräsentation (R1,2) des Untersuchungsbereichs des Untersuchungsobjekts in einem ersten Zustand, wobei die mindestens eine Eingabe-Repräsentation (R1,2) den Untersuchungsbereich vor und/oder nach der Applikation einer ersten Menge eines Kontrastmittels repräsentiert,
   - Bereitstellen eines trainieren Modells ($MLM^t$) des maschinellen Lernens,

      • wobei das trainierte Modell ($MLM^t$) des maschinellen Lernens anhand von Trainingsdaten (TD) trainiert worden ist, auf Basis mindestens einer Eingabe-Repräsentation (R1, R2) eines Untersuchungsbereichs eines Untersuchungsobjekts in einem ersten Zustand eine synthetische Repräsentation (SR) des Untersuchungsbereichs in einem zweiten Zustand zu erzeugen,
      • wobei die Trainingsdaten (TD) für jedes Untersuchungsobjekt einer Vielzahl von Untersuchungsobjekten i) eine Eingabe-Repräsentation (R1) des Untersuchungsbereichs, ii) eine Ziel-Repräsentation (TR) und iii) eine transformierte Ziel-Repräsentation ($TR^T$) umfassen,
      • wobei die transformierte Ziel-Repräsentation ($TR^T$) zumindest einen Teil des Untersuchungsbereichs des Untersuchungsobjekts im Frequenzraum repräsentiert, falls die Ziel-Repräsentation (TR) den Untersuchungsbereichs des Untersuchungsobjekts im Ortsraum repräsentiert, oder im Ortsraum repräsentiert, falls die Ziel-Repräsentation (TR) den Untersuchungsbereich des Untersuchungsobjekts im Frequenzraum repräsentiert,
      • wobei das Modell ($MLM^t$) des maschinellen Lernens trainiert worden ist, für jedes Untersuchungsobjekt, Abweichungen zwischen i) zumindest einem Teil der synthetischen Repräsentation (SR) und zumindest einem Teil der Ziel-Repräsentation (TR) und ii) zwischen zumindest einem Teil einer transformierten synthetischen Repräsentation ($SR^T$) und zumindest einem Teil der transformierten ZielRepräsentation ($TR^T$) zu minimieren,

   - Eingeben der mindestens einen empfangenen Eingabe-Repräsentation (R1,2) des Untersuchungsbereichs des Untersuchungsobjekts in das trainierte Modell ($MLM^t$) des maschinellen Lernens,
   - Empfangen einer synthetischen Repräsentation (SR) des Untersuchungsbereichs des Untersuchungsobjekts in dem zweiten Zustand von dem Modell ($MLM^t$) des maschinellen Lernens, wobei die synthetische Repräsentation (SR*) den Untersuchungsbereich nach der Applikation einer zweiten Menge des Kontrastmittels repräsentiert, wobei die zweite Menge verschieden von der ersten Menge ist,
   - Ausgeben und/oder Speichern der synthetischen Repräsentation (SR) und/oder Übermitteln der synthetischen Repräsentation (SR) an ein separates Computersystem.

11. Verfahren gemäß Anspruch 10, wobei das trainierte Modell ($MLM^t$) des maschinellen Lernens in einem Verfahren gemäß einem der Ansprüche 1 bis 9 trainiert worden ist.

12. Computersystem (10) umfassend

   • eine Empfangseinheit (11),
   • eine Steuer- und Recheneinheit (12) und
   • eine Ausgabeeinheit (13),

      - wobei die Steuer- und Recheneinheit (12) konfiguriert ist, die Empfangseinheit (11) zu veranlassen, mindestens eine Eingabe-Repräsentation (R1,2) eines Untersuchungsbereichs eines neuen Untersuchungsobjekts in einem ersten Zustand zu empfangen, wobei die mindestens eine Eingabe-Repräsentation (R1,2) den Untersuchungsbereich vor und/oder nach der Applikation einer ersten Menge eines Kontrastmittels repräsentiert,
      - wobei die Steuer- und Recheneinheit (12) konfiguriert ist, die empfangene mindestens eine Eingabe-Repräsentation (R1,2) in ein trainiertes Modell ($MLM^t$) des maschinellen Lernens einzugeben,

o wobei das trainierte Modell (MLM$^t$) des maschinellen Lernens anhand von Trainingsdaten (TD) trainiert worden ist, auf Basis mindestens einer Eingabe-Repräsentation (R1, R2) eines Untersuchungsbereichs eines Untersuchungsobjekts in einem ersten Zustand eine synthetische Repräsentation (SR) des Untersuchungsbereichs in einem zweiten Zustand zu erzeugen, o wobei die Trainingsdaten (TD) für jedes Untersuchungsobjekt einer Vielzahl von Untersuchungsobjekten i) eine Eingabe-Repräsentation (R1) des Untersuchungsbereichs, ii) eine Ziel-Repräsentation (TR) und iii) eine transformierte Ziel-Repräsentation (TR$^T$) umfassen, o wobei die transformierte Ziel-Repräsentation (TR$^T$) zumindest einen Teil des Untersuchungsbereichs des Untersuchungsobjekts im Frequenzraum repräsentiert, falls die Ziel-Repräsentation (TR) den Untersuchungsbereichs des Untersuchungsobjekts im Ortsraum repräsentiert, oder im Ortsraum repräsentiert, falls die Ziel-Repräsentation (TR) den Untersuchungsbereich des Untersuchungsobjekts im Frequenzraum repräsentiert, o wobei das Modell (MLM$^t$) des maschinellen Lernens trainiert worden ist, für jedes Untersuchungsobjekt, Abweichungen zwischen i) zumindest einem Teil der synthetischen Repräsentation (SR) und zumindest einem Teil der Ziel-Repräsentation (TR) und ii) zwischen zumindest einem Teil einer transformierten synthetischen Repräsentation (SR$^T$) und zumindest einem Teil der transformierten Ziel-Repräsentation (TR$^T$) zu minimieren,

- wobei die Steuer- und Recheneinheit (12) konfiguriert ist, von dem Modell (MLM$^t$) des maschinellen Lernens eine synthetische Repräsentation (SR) des Untersuchungsbereichs des neuen Untersuchungsobjekts in dem zweiten Zustand zu empfangen, wobei die synthetische Repräsentation (SR) den Untersuchungsbereich nach der Applikation einer zweiten Menge des Kontrastmittels repräsentiert, wobei die zweite Menge verschieden von der ersten Menge ist,
- wobei die Steuer- und Recheneinheit (12) konfiguriert ist, die Ausgabeeinheit (13) zu veranlassen, die synthetische Repräsentation (SR) auszugeben und/oder zu speichern und/oder an ein separates Computersystem zu übermitteln.

13. Computerprogrammprodukt umfassend ein Computerprogramm (40), das in einen Arbeitsspeicher (22) eines Computersystems (1) geladen werden kann und dort das Computersystem (1) dazu veranlasst, folgende Schritte ausführen:

- Empfangen mindestens einer Eingabe-Repräsentation (R1,2) des Untersuchungsbereichs des Untersuchungsobjekts in einem ersten Zustand, wobei die mindestens eine Eingabe-Repräsentation (R1,2) den Untersuchungsbereich vor und/oder nach der Applikation einer ersten Menge eines Kontrastmittels repräsentiert,
- Bereitstellen eines trainieren Modells (MLM$^t$) des maschinellen Lernens,

• wobei das trainierte Modell (MLM$^t$) des maschinellen Lernens anhand von Trainingsdaten (TD) trainiert worden ist, auf Basis mindestens einer Eingabe-Repräsentation (R1, R2) eines Untersuchungsbereichs eines Untersuchungsobjekts in einem ersten Zustand eine synthetische Repräsentation (SR) des Untersuchungsbereichs in einem zweiten Zustand zu erzeugen, • wobei die Trainingsdaten (TD) für jedes Untersuchungsobjekt einer Vielzahl von Untersuchungsobjekten i) eine Eingabe-Repräsentation (R1) des Untersuchungsbereichs, ii) eine Ziel-Repräsentation (TR) und iii) eine transformierte Ziel-Repräsentation (TR$^T$) umfassen, • wobei die transformierte Ziel-Repräsentation (TR$^T$) zumindest einen Teil des Untersuchungsbereichs des Untersuchungsobjekts im Frequenzraum repräsentiert, falls die Ziel-Repräsentation (TR) den Untersuchungsbereichs des Untersuchungsobjekts im Ortsraum repräsentiert, oder im Ortsraum repräsentiert, falls die Ziel-Repräsentation (TR) den Untersuchungsbereich des Untersuchungsobjekts im Frequenzraum repräsentiert, • wobei das Modell (MLM$^t$) des maschinellen Lernens trainiert worden ist, für jedes Untersuchungsobjekt, Abweichungen zwischen i) zumindest einem Teil der synthetischen Repräsentation (SR) und zumindest einem Teil der Ziel-Repräsentation (TR) und ii) zwischen zumindest einem Teil einer transformierten synthetischen Repräsentation (SR$^T$) und zumindest einem Teil der transformierten ZielRepräsentation (TR$^T$) zu minimieren,

- Eingeben der mindestens einen empfangenen Eingabe-Repräsentation (R1,2) des Untersuchungsbereichs des Untersuchungsobjekts in das trainierte Modell (MLM$^t$) des maschinellen Lernens,
- Empfangen einer synthetischen Repräsentation (SR) des Untersuchungsbereichs des Untersuchungsobjekts in dem zweiten Zustand von dem Modell (MLM$^t$) des maschinellen Lernens, wobei die synthetische Repräsentation (SR) den Untersuchungsbereich nach der Applikation einer zweiten Menge des Kontrastmittels repräsentiert, wobei die zweite Menge verschieden von der ersten Menge ist,
- Ausgeben und/oder Speichern der synthetischen Repräsentation (SR) und/oder Übermitteln der synthetischen Repräsentation (SR) an ein separates Computersystem.

**Claims**

1. Method for training a machine-learning model (MLM) for producing synthetic radiological images based on measured radiological images, wherein the method comprises:

- receiving and/or providing training data (TD), the training data for each examination object of a multiplicity of examination objects comprising a set of input data and target data, wherein each set comprises

o at least one input representation (R1, R2) of an examination region of the examination object in a first state as input data and
◦ a target representation (TR) of the examination region of the examination object in a second state and also a transformed target representation (TR$^T$) as target data, wherein the transformed target representation (TR$^T$) represents at least one part of the examination region of the examination object
o in frequency space, if the target representation (TR) represents the examination region of the examination object in real space, or
o in real space, if the target representation (TR) represents the examination region of the examination object in frequency space,

- training a machine-learning model (MLM), the machine-learning model (MLM) being configured to produce a synthetic representation (SR) of the examination region of the examination object on the basis of at least one input representation (R1, R2) and model parameters (MP), with the at least one input representation (R1, R2) representing the examination region before and/or after the application of a first amount of a contrast agent, the synthetic representation (SR) representing the examination region after the application of a second amount of contrast agent, and the first amount differing from the second amount,
wherein training for each examination object of the multiplicity of examination objects comprises:

o supplying the at least one input representation (R1, R2) to the machine-learning model (MLM),
o receiving a synthetic representation (SR) of the examination region of the examination object from the machine-learning model (MLM),
o producing and/or receiving a transformed synthetic representation (SR$^T$) on the basis of and/or relating to the synthetic representation (SR), the transformed synthetic representation (SR$^T$) representing at least one part of the examination region of the examination object

▪ in frequency space, if the synthetic representation (SR) represents the examination region of the examination object in real space, or
▪ in real space, if the synthetic representation (SR) represents the examination region of the examination object in frequency space,

o quantifying the deviations i) between at least one part of the synthetic representation (SR) and at least one part of the target representation (TR) and ii) between at least one part ($SR^{T,P}$) of the transformed synthetic representation (SR$^T$) and at least one part ($TR^{T,P}$) of the transformed target representation (TR$^T$) by means of an error function (L),
o modifying model parameters (MP) with regards to reduced deviations,

- outputting and/or storing the trained machine-learning model (MLM$^t$) and/or model parameters (MP) and/or transmitting the trained machine-learning model (MLM$^t$) and/or model parameters (MP) to a separate computer system.

2. Method according to Claim 1,

> wherein receiving and/or providing training data (TD) comprises:
>
>> - producing a partial transformed target representation ($TR^{T,P}$), the partial transformed target representation ($TR^{T,P}$) being reduced to one or more parts of the transformed target representation ($TR^T$),
>
> wherein the production and/or reception of a transformed synthetic representation ($SR^T$) on the basis of and/or relating to the synthetic representation (SR) comprises:
>
>> - producing a partial transformed synthetic representation ($SR^{T,P}$), the partial transformed synthetic representation ($SR^{T,P}$) being reduced to one or more parts of the transformed synthetic representation ($SR^T$),
>
> wherein the quantification of the deviations between the transformed synthetic representation ($SR^T$) and the transformed target representation ($TR^T$) comprises:
>
>> - quantifying the deviations between the partial transformed synthetic representation ($SR^{T,P}$) and the partial transformed target representation ($TR^{T,P}$).

3. Method according to Claim 1 or 2,
   wherein training for each examination object of the multiplicity of examination objects comprises:

> o supplying the at least one input representation (R1, R2) to the machine-learning model (MLM),
> o receiving the synthetic representation (SR) and a first transformed synthetic representation ($SR^T$) of the examination region of the examination object from the machine-learning model (MLM), the first transformed synthetic representation ($SR^T$) representing at least one part of the examination region of the examination object
>
>> ▪ in frequency space, if the synthetic representation (SR) represents the examination region of the examination object in real space, or
>> ▪ in real space, if the synthetic representation (SR) represents the examination region of the examination object in frequency space,
>
> o producing a second transformed synthetic representation ($SR^{T*}$) on the basis of the synthetic representation (SR) by means of a transformation (T), the second transformed synthetic representation ($SR^{T*}$) representing at least one part of the examination region of the examination object

>> ▪ in frequency space, if the synthetic representation (SR) represents the examination region of the examination object in real space, or
>> ▪ in real space, if the synthetic representation (SR) represents the examination region of the examination object in frequency space,
>
>> - quantifying the deviations i) between at least one part of the synthetic representation (SR) and at least one part of the target representation (TR), ii) between at least one part of the first transformed synthetic representation ($SR^T$) and at least one part of the transformed target representation (TR*), and iii) between at least one part of the first transformed synthetic representation ($SR^T$) and at least one part of the second transformed synthetic representation ($SR^{T*}$) by means of an error function (L),
>> - modifying model parameters (MP) with regards to reduced deviations.

4. Method according to any of Claims 1 to 3,

> wherein the machine-learning model (MLM) comprises a first machine-learning model (MLM1) and a second machine-learning model (MLM2),
> wherein the first machine-learning model (MLM1) is configured to produce a synthetic representation (SR) of the examination region of the examination object on the basis of at least one input representation (R1) and model parameters (MP1),
> wherein the second machine-learning model (MLM2) is configured to reconstruct the input representation (R1) on the basis of the synthetic representation (SR) of the examination region of the examination object,
> wherein training for each examination object of the multiplicity of examination objects comprises:
>
>> o producing a transformed input representation (R2) on the basis of the input representation (R1) by means of a transformation (T), the transformed input representation (R2) representing at least one part of the examination region of the examination object

▪ in frequency space, if the input representation (R1) represents the examination region of the examination object in real space, or
▪ in real space, if the input representation (R1) represents the examination region of the examination object in frequency space,

o supplying the at least one input representation (R1) to the first machine-learning model (MLM1),
o receiving a synthetic representation (SR) of the examination region of the examination object from the first machine-learning model (MLM1),
o producing and/or receiving a transformed synthetic representation (SR$^T$) on the basis of and/or relating to the synthetic representation (SR), the transformed synthetic representation (SR$^T$) representing at least one part of the examination region of the examination object

▪ in frequency space, if the synthetic representation (SR) represents the examination region of the examination object in real space, or
▪ in real space, if the synthetic representation (SR) represents the examination region of the examination object in frequency space,

o supplying the synthetic representation (SR) and/or the transformed synthetic representation (SR$^T$) to the second machine-learning model (MLM2),
o receiving a predicted input representation (R1*) from the second machine-learning model (MLM2),
o producing and/or receiving a transformed predicted input representation (R2*) on the basis of and/or relating to the predicted input representation (R1*), the transformed predicted input representation (R2*) representing at least one part of the examination region of the examination object

▪ in frequency space, if the predicted input representation (R1*) represents the examination region of the examination object in real space, or
▪ in real space, if the predicted input representation (R1*) represents the examination region of the examination object in frequency space,

o quantifying the deviations i) between at least one part of the synthetic representation (SR) and at least one part of the target representation (TR), ii) between at least one part of the transformed synthetic representation (SR$^T$) and at least one part of the transformed target representation (TR$^T$), iii) between at least one part of the input representation (R1) and at least one part of the predicted input representation (R1*) and iv) between at least one part of the transformed input representation (R2) and at least one part of the transformed predicted input representation (R2*) by means of an error function,
o modifying model parameters (MP1, MP2) with regards to reduced deviations.

5. Method according to any of Claims 1 to 4, wherein the first amount is less than the second amount.

6. Method according to any of Claims 1 to 5, wherein the examination region is a liver or a part of a liver of a human being.

7. Method according to any of Claims 1 to 6, wherein each of the at least one input representation (R1, R2) is a representation of the examination region in real space, the target representation (TR) is a representation of the examination region in real space and the synthetic representation (SR) is a representation of the examination region in real space.

8. Method according to any of Claims 2 to 6, wherein the partial transformed synthetic representation (SR$^{T,P}$) represents the examination region in frequency space, the partial transformed synthetic representation (SR$^{T,P}$) being reduced to a frequency range of the transformed synthetic representation (SR$^T$), with contrast information being encoded in the frequency range.

9. Method according to any of Claims 2 to 6, wherein the partial transformed synthetic representation (SR$^{T,P}$) represents the examination region in frequency space, the partial transformed synthetic representation (SR$^{T,P}$) being reduced to a frequency range of the transformed synthetic representation (SR$^T$), with information relating to fine structures being encoded in the frequency range.

10. Computer-implemented method for producing a synthetic representation of an examination region of an examination object, wherein the method comprises:

- receiving at least one input representation (R1,2) of the examination region of the examination object in a first state, the at least one input representation (R1,2) representing the examination region before and/or after the application of a first amount of a contrast agent,
- providing a trained machine-learning model (MLM$^t$),

• wherein the trained machine-learning model (MLM$^t$) has been trained on the basis of training data (TD) to produce a synthetic representation (SR) of the examination region in a second state on the basis of at least one input representation (R1, R2) of an examination region of an examination object in a first state,
• wherein the training data (TD) for each examination object of a multiplicity of examination objects comprise i) an input representation (R1) of the examination region, ii) a target representation (TR) and iii) a transformed target representation (TR$^T$),
• wherein the transformed target representation (TR$^T$) represents at least one part of the examination region of the examination object in frequency space, if the target representation (TR) represents the examination region of the examination object in real space, or in real space, if the target representation (TR) represents the examination region of the examination object in frequency space,
• wherein the machine-learning model (MLM$^t$) has been trained for each examination object to minimize deviations between i) at least one part of the synthetic representation (SR) and at least one part of the target representation (TR) and ii) between at least one part of a transformed synthetic representation (SR$^T$) and at least one part of the transformed target representation (TR$^T$),

- inputting the at least one received input representation (R1,2) of the examination region of the examination object into the trained machine-learning model (MLM$^t$),
- receiving a synthetic representation (SR) of the examination region of the examination object in the second state from the machine-learning model (MLM$^t$), the synthetic representation (SR*) representing the examination region after the application of a second amount of contrast agent, with the second amount differing from the first amount,
- outputting and/or storing the synthetic representation (SR) and/or transmitting the synthetic representation (SR) to a separate computer system.

11. Method according to Claim 10, wherein the trained machine-learning model (MLM$^t$) has been trained in a method according to any of Claims 1 to 9.

12. Computer system (10) comprising

• a receiving unit (11),
• a control and calculation unit (12) and
• an output unit (13),

- wherein the control and calculation unit (12) is configured to cause the receiving unit (11) to receive at least one input representation (R1,2) of an examination region of a new examination object in a first state, the at least one input representation (R1,2) representing the examination region before and/or after the application of a first amount of a contrast agent,
- wherein the control and calculation unit (12) is configured to input the received at least one input representation (R1,2) into a trained machine-learning model (MLM$^t$),

o wherein the trained machine-learning model (MLM$^t$) has been trained on the basis of training data (TD) to produce a synthetic representation (SR) of the examination region in a second state on the basis of at least one input representation (R1, R2) of an examination region of an examination object in a first state,
o wherein the training data (TD) for each examination object of a multiplicity of examination objects comprise i) an input representation (R1) of the examination region, ii) a target representation (TR) and iii) a transformed target representation (TR$^T$),
o wherein the transformed target representation (TR$^T$) represents at least one part of the examination region of the examination object in frequency space, if the target representation (TR) represents the examination region of the examination object in real space, or in real space, if the target representation (TR) represents the examination region of the examination object in frequency space,
o wherein the machine-learning model (MLM$^t$) has been trained for each examination object to minimize deviations between i) at least one part of the synthetic representation (SR) and at least one part of the target representation (TR) and ii) between at least one part of a transformed synthetic representation (SR$^T$) and at least one part of the transformed target representation (TR$^T$),

- wherein the control and calculation unit (12) is configured to receive from the machine-learning model (MLM$^t$) a synthetic representation (SR) of the examination region of the new examination object in the second state, the synthetic representation (SR) representing the examination region after the application of a second amount of contrast agent, the second amount differing from the first amount,
- wherein the control and calculation unit (12) is configured to cause the output unit (13) to output and/or store the synthetic representation (SR) and/or transmit the latter to a separate computer system.

13. Computer program product comprising a computer program (40) that can be loaded into a working memory (22) of a computer system (1), where it causes the computer system (1) to execute the following steps:

- receiving at least one input representation (R1,2) of the examination region of the examination object in a first state, the at least one input representation (R1,2) representing the examination region before and/or after the application of a first amount of a contrast agent,
- providing a trained machine-learning model (MLM$^t$),

• wherein the trained machine-learning model (MLM$^t$) has been trained on the basis of training data (TD) to produce a synthetic representation (SR) of the examination region in a second state on the basis of at least one input representation (R1, R2) of an examination region of an examination object in a first state,
• wherein the training data (TD) for each examination object of a multiplicity of examination objects comprise i) an input representation (R1) of the examination region, ii) a target representation (TR) and iii) a transformed target representation (TR$^T$),
• wherein the transformed target representation (TR$^T$) represents at least one part of the examination region of the examination object in frequency space, if the target representation (TR) represents the examination region of the examination object in real space, or in real space, if the target representation (TR) represents the examination region of the examination object in frequency space,
• wherein the machine-learning model (MLM$^t$) has been trained for each examination object to minimize deviations between i) at least one part of the synthetic representation (SR) and at least one part of the target representation (TR) and ii) between at least one part of a transformed synthetic representation (SR$^T$) and at least one part of the transformed target representation (TR$^T$),

- inputting the at least one received input representation (R1,2) of the examination region of the examination object into the trained machine-learning model (MLM$^t$),
- receiving a synthetic representation (SR) of the examination region of the examination object in the second state from the machine-learning model (MLM$^t$), the synthetic representation (SR) representing the examination region after the application of a second amount of contrast agent, with the second amount differing from the first amount,
- outputting and/or storing the synthetic representation (SR) and/or transmitting the synthetic representation (SR) to a separate computer system.

**Revendications**

1. Procédé pour l'entraînement d'un modèle (MLM) d'apprentissage automatique pour générer des images radiologiques synthétiques sur la base d'images radiologiques mesurées, le procédé comprenant :

- la réception et/ou la fourniture de données d'entraînement (TD), les données d'entraînement (TD), pour chaque objet d'examen d'une pluralité d'objets d'examen, comprenant un ensemble de données d'entrée et de données cibles,
chaque ensemble comprenant

◦ au moins une représentation d'entrée (R1, R2) d'une zone d'examen de l'objet d'examen dans un premier état en tant que données d'entrée, et
o une représentation cible (TR) de la zone d'examen de l'objet d'examen dans un deuxième état ainsi qu'une représentation cible transformée (TR$^T$) en tant que données cibles,
la représentation cible transformée (TR$^T$) représentant au moins une partie de la zone d'examen de l'objet d'examen o dans le domaine fréquentiel si la représentation cible (TR) représente la zone d'examen de l'objet d'examen dans le domaine spatial, ou

o dans le domaine spatial si la représentation cible (TR) représente la zone d'examen de l'objet d'examen dans le domaine fréquentiel,

- l'entraînement d'un modèle (MLM) d'apprentissage automatique, le modèle (MLM) d'apprentissage automatique étant configuré pour générer, sur la base d'au moins une représentation d'entrée (R1, R2) et de paramètres de modèle (MP), une représentation synthétique (SR) de la zone d'examen de l'objet d'examen, ladite au moins une représentation d'entrée (R1, R2) représentant la zone d'examen après l'application d'une quantité d'un agent de contraste, et la représentation synthétique (SR) représentant la zone d'examen après l'application de la quantité de l'agent de contraste, la deuxième quantité étant différente de la deuxième quantité, l'entraînement, pour chaque objet d'examen de la pluralité d'objets d'examen, comprenant :

o la fourniture de ladite au moins une représentation d'entrée (R1, R2) au modèle (MLM) d'apprentissage automatique,
o la réception en provenance du modèle (MLM) d'apprentissage automatique d'une représentation synthétique (SR) de la zone d'examen de l'objet d'examen,
o la génération et/ou la réception d'une représentation synthétique transformée (SR$^T$) basée sur, et/ou en relation avec, la représentation synthétique (SR), la représentation synthétique transformée (SR$^T$) représentant au moins une partie de la zone d'examen de l'objet d'examen

■ dans le domaine fréquentiel, si la représentation synthétique (SR) représente la zone d'examen de l'objet d'examen dans le domaine spatial, ou
■ dans le domaine spatial, si la représentation synthétique (SR) représente la zone d'examen de l'objet d'examen dans le domaine fréquentiel,

o la quantification des écarts i) entre au moins une partie de la représentation synthétique (SR) et au moins une partie de la représentation cible (TR) et ii) entre au moins une partie (SR$^{T,P}$) de la représentation synthétique transformée (SR$^T$) et au moins une partie (TR$^{T,P}$) de la représentation cible transformée (TR$^T$), au moyen d'une fonction d'erreur (L),

- la modification de paramètres de modèle (MP) en vue de réduire les écarts,
- la fourniture en sortie et/ou la mémorisation du modèle entraîné (MLM$^t$) d'apprentissage automatique et/ou des paramètres de modèle (MP) et/ou la transmission du modèle entraîné (MLM$^t$) d'apprentissage automatique et/ou des paramètres de modèle (MP) à un système d'ordinateur distinct.

2. Procédé selon la revendication 1,

dans lequel la réception et/ou la fourniture de données d'entraînement (TD) comprend :

- la génération d'une représentation cible transformée partielle (TR$^{T,P}$), la représentation cible transformée partielle (TR$^{T,P}$) étant réduite à une ou plusieurs parties de la représentation cible transformée (TR$^T$),

la génération et/ou la réception d'une représentation synthétique transformée (SRT) basée sur, et/ou en relation avec, la représentation synthétique (SR) comprenant :

- la génération d'une représentation synthétique transformée partielle (SR$^{T,P}$), la représentation synthétique transformée partielle (SR$^{T,P}$) étant réduite à une partie ou plusieurs parties de la représentation synthétique transformée (SR$^T$),

la quantification des écarts entre la représentation synthétique transformée (SR$^T$) et la représentation cible transformée (TR$^T$) comprenant :

- la quantification des écarts entre la représentation synthétique transformée partielle (SR$^{T,P}$) et la représentation cible transformée partielle (TR$^{T,P}$).

3. Procédé selon la revendication 1 ou la revendication 2,
dans lequel l'entraînement comprend, pour chaque objet d'examen de la pluralité d'objets d'examen :

o la fourniture de ladite au moins une représentation d'entrée (R1, R2) au modèle (MLM) d'apprentissage automatique,
o la réception en provenance du modèle (MLM) d'apprentissage automatique de la représentation synthétique (SR) et d'une première représentation synthétique transformée (SR$^T$) de la zone d'examen de l'objet d'examen, la première représentation synthétique transformée (SR$^T$) représentant au moins une partie de la zone d'examen de l'objet d'examen

■ dans le domaine fréquentiel, si la représentation synthétique (SR) représente la zone d'examen de l'objet d'examen dans le domaine spatial, ou

■ dans le domaine spatial, si la représentation synthétique (SR) représente la zone d'examen de l'objet d'examen dans le domaine fréquentiel,

o la génération d'une deuxième représentation synthétique transformée (SR$T$*) sur la base de la représentation synthétique (SR) au moyen d'une transformation (T), la deuxième représentation synthétique transformée (SR$T$*) représentant au moins une partie de la zone d'examen de l'objet d'examen

■ dans le domaine fréquentiel, si la représentation synthétique (SR) représente la zone d'examen de l'objet d'examen dans le domaine spatial, ou

■ dans le domaine spatial, si la représentation synthétique (SR) représente la zone d'examen de l'objet d'examen dans le domaine fréquentiel,

- la quantification des écarts i) entre au moins une partie de la représentation synthétique (SR) et au moins une partie de la représentation cible (TR), ii) entre au moins une partie de la première représentation synthétique transformée (SR$^T$) et au moins une partie de la représentation cible transformée (TR*), et iii) entre au moins une partie de la première représentation synthétique transformée (SR$^T$) et au moins une partie de la deuxième représentation synthétique transformée (SR$T$*) au moyen d'une fonction d'erreur (L),
- la modification de paramètres de modèle (MP) en vue de réduire les écarts.

4. Procédé selon l'une des revendications 1 à 3,

dans lequel le modèle (MLM) d'apprentissage automatique comprend un premier modèle (MLM1) d'apprentissage automatique et un deuxième modèle (MLM2) d'apprentissage automatique,
le premier modèle (MLM1) d'apprentissage automatique étant configuré pour générer, sur la base d'au moins une représentation d'entrée (R1) et de paramètres de modèle (MP1), une représentation synthétique (SR) de la zone d'examen de l'objet d'examen,
le deuxième modèle (MLM2) d'apprentissage automatique étant configuré pour reconstruire, sur la base de la représentation synthétique (SR) de la zone d'examen de l'objet d'examen, la représentation d'entrée (R1),
dans lequel l'entraînement comprend, pour chaque objet d'examen de la pluralité d'objets d'examen :

o la génération d'une représentation d'entrée transformée (R2) sur la base de la représentation d'entrée (R1) au moyen d'une transformation (T), la représentation d'entrée transformée (R2) représentant au moins une partie de la zone d'examen de l'objet d'examen

■ dans le domaine fréquentiel, si la représentation d'entrée (R1) représente la zone d'examen de l'objet d'examen dans le domaine spatial, ou

■ dans le domaine spatial, si la représentation d'entrée (R1) représente la zone d'examen de l'objet d'examen dans le domaine fréquentiel,

o l'envoi de ladite au moins une représentation d'entrée (R1) au premier modèle (MLM1) d'apprentissage automatique,
o la réception en provenance du premier modèle (MLM1) d'apprentissage automatique d'une représentation synthétique (SR) de la zone d'examen de l'objet d'examen,
o la génération et/ou la réception d'une représentation synthétique transformée (SR$^T$) basée sur, et/ou en relation avec, la représentation synthétique (SR), la représentation synthétique transformée (SR$^T$) représentant au moins une partie de la zone d'examen de l'objet d'examen

■ dans le domaine fréquentiel, si la représentation synthétique (SR) représente la zone d'examen de l'objet d'examen dans le domaine spatial, ou

■ dans le domaine spatial, si la représentation synthétique (SR) représente la zone d'examen de l'objet d'examen dans le domaine fréquentiel,

o l'envoi de la représentation synthétique (SR) et/ou de la représentation synthétique transformée (SR$^T$) au deuxième modèle (MLM2) d'apprentissage automatique,
o la réception d'une représentation d'entrée prédite (R1*) en provenance du deuxième modèle (MLM2) d'apprentissage automatique,
o la génération et/ou la réception d'une représentation d'entrée prédite transformée (R2*) basée sur, et/ou en relation avec, la représentation d'entrée prédite (R1*), la représentation d'entrée prédite transformée (R2*) représentant au moins une partie de la zone d'examen de l'objet d'examen

- ▪ dans le domaine fréquentiel, si la représentation d'entrée prédite (R1*) représente la zone d'examen de l'objet d'examen dans le domaine spatial, ou
- ▪ dans le domaine spatial, si la représentation d'entrée prédite (R1*) représente la zone d'examen de l'objet d'examen dans le domaine fréquentiel,

o la quantification des écarts i) entre au moins une partie de la représentation synthétique (SR) et au moins une partie de la représentation cible (TR), ii) entre au moins une partie de la représentation synthétique transformée ($SR^T$) et au moins une partie de la représentation cible transformée ($TR^T$), iii) entre au moins une partie de la représentation d'entrée (R1) et au moins une partie de la représentation d'entrée prédite (R1*) et iv) entre au moins une partie de la représentation d'entrée transformée (R2) et au moins une partie de la représentation d'entrée prédite transformée (R2*) au moyen d'une fonction d'erreur,
o la modification de paramètres de modèle (MP1, MP2) en vue de réduire les écarts.

5. Procédé selon l'une des revendications 1 à 4, dans lequel la première quantité est inférieure à la deuxième quantité.

6. Procédé selon l'une des revendications 1 à 5, dans lequel la zone d'examen est un foie ou une partie d'un foie d'un être humain.

7. Procédé selon l'une des revendications 1 à 6, dans lequel chacune desdites au moins une représentation d'entrée (R1, R2) est une représentation de la zone d'examen dans le domaine spatial, la représentation cible (TR) est une représentation de la zone d'examen dans le domaine spatial et la représentation synthétique (SR) est une représentation de la zone d'examen dans le domaine spatial.

8. Procédé selon l'une des revendications 2 à 6, dans lequel la représentation synthétique transformée partielle ($SR^{T,P}$) représente la zone d'examen dans le domaine fréquentiel, la représentation synthétique transformée partielle ($SR^{T,P}$) étant réduite à une plage de fréquences de la représentation synthétique transformée ($SR^T$), et des informations de contraste étant codées dans la plage de fréquences.

9. Procédé selon l'une des revendications 2 à 6, dans lequel la représentation synthétique transformée partielle ($SR^{T,P}$) représente la zone d'examen dans le domaine fréquentiel, la représentation synthétique transformée partielle ($SR^{T,P}$) étant réduite à une plage de fréquences de la représentation synthétique transformée ($SR^T$), et des informations sur les structures fines étant codées dans la plage de fréquences.

10. Procédé mis en œuvre par ordinateur pour la génération d'une représentation synthétique d'une zone d'examen d'un objet d'examen, le procédé comprenant :

- la réception d'au moins une représentation d'entrée (R1,2) de la zone d'examen de l'objet d'examen dans un premier état, ladite au moins une représentation d'entrée (R1,2) représentant la zone d'examen avant et/ou après l'application d'une première quantité d'un agent de contraste,
- la fourniture d'un modèle entraîné ($MLM^t$) d'apprentissage automatique,

• le modèle entraîné ($MLM^t$) d'apprentissage automatique ayant été entraîné à partir de données d'entraînement (TD) pour générer, sur la base d'au moins une représentation d'entrée (R1, R2) une zone d'examen d'un objet d'examen dans un premier état, une représentation synthétique (SR) de la zone d'examen dans un deuxième état,
• les données d'entraînement (TD) comprenant, pour chaque objet d'examen d'une pluralité d'objets d'examen, i) une représentation d'entrée (R1) de la zone d'examen, ii) une représentation cible (TR) et iii) une représentation cible transformée ($TR^T$),
• la représentation cible transformée ($TR^T$) représentant au moins une partie de la zone d'examen de l'objet d'examen dans le domaine fréquentiel, si la représentation cible (TR) représente la zone d'examen de l'objet d'examen dans le domaine spatial, ou la représentant dans le domaine spatial, si la représentation cible (TR) représente la zone d'examen de l'objet d'examen dans le domaine fréquentiel,
• le modèle ($MLM^t$) d'apprentissage automatique ayant été entraîné, pour chaque objet d'examen, de façon à rendre minimaux les écarts entre i) au moins une partie de la représentation synthétique (SR) et au moins une partie de la représentation cible (TR) et ii) entre au moins une partie d'une représentation synthétique transformée (SR$^T$) et au moins une partie de la représentation cible transformée (TR$^T$),

- la fourniture en entrée au modèle entraîné (MLM$^t$) d'apprentissage automatique de ladite au moins une représentation d'entrée reçue (R1,2) de la zone d'examen de l'objet d'examen,
- la réception, en provenance du modèle (MLM$^t$) d'apprentissage automatique, d'une représentation synthétique (SR) de la zone d'examen de l'objet d'examen dans le deuxième état, la représentation synthétique (SR*) représentant la zone d'examen après l'application de la quantité de l'agent de contraste, la deuxième quantité étant différente de la première quantité,
- la fourniture en sortie, à un système d'ordinateur distinct, et/ou la mémorisation de la représentation synthétique (SR*) et/ou la transmission de la représentation synthétique (SR*).

11. Procédé selon la revendication 10, dans lequel le modèle entraîné (MLM$^t$) d'apprentissage automatique a été entraîné par un procédé selon l'une des revendications 1 à 9.

12. Système d'ordinateur (10) comprenant

• une unité de réception (11),
• une unité de commande et de calcul (12), et
• une unité de sortie (13),

- l'unité de commande et de calcul (12) étant configurée pour amener l'unité de réception (11) à recevoir au moins une représentation d'entrée (R1,2) d'une zone d'examen d'un nouvel objet d'examen dans un premier état, ladite au moins une représentation d'entrée (R1,2) représentant la zone d'examen avant et/ou après l'application d'une première quantité d'un agent de contraste,
- l'unité de commande et de calcul (12) étant configurée pour fournir en entrée, à un modèle entraîné (MLM$^t$) d'apprentissage automatique, la représentation d'entrée (R1,2) reçue,

o le modèle entraîné (MLM$^t$) d'apprentissage automatique ayant été entraîné à partir de données d'entraînement (TD) pour générer, sur la base d'au moins une représentation d'entrée (R1, R2) d'une zone d'examen d'un objet d'examen dans un premier état, une représentation synthétique (SR) de la zone d'examen dans un deuxième état,

• les données d'entraînement (TD) comprenant, pour chaque objet d'examen d'une pluralité d'objets d'examen,

i) une représentation d'entrée (R1) de la zone d'examen,
ii) une représentation cible (TR) et iii) une représentation cible transformée (TR$^T$),

o la représentation cible transformée (TR$^T$) représentant au moins une partie de la zone d'examen de l'objet d'examen dans le domaine fréquentiel, si la représentation cible (TR) représente la zone d'examen de l'objet d'examen dans le domaine spatial, ou la représentant dans le domaine spatial, si la représentation cible (TR) représente la zone d'examen de l'objet d'examen dans le domaine fréquentiel,
o le modèle (MLM$^t$) d'apprentissage automatique ayant été entraîné, pour chaque objet d'examen, de façon à rendre minimaux les écarts entre i) au moins une partie de la représentation synthétique (SR) et au moins une partie de la représentation cible (TR) et ii) entre au moins une partie d'une représentation synthétique transformée (SR$^T$) et au moins une partie de la représentation cible transformée (TR$^T$),

- l'unité de commande et de calcul (12) étant configurée pour recevoir, en provenance du modèle (MLM$^t$) d'apprentissage automatique, une représentation synthétique (SR) de la zone d'examen du nouvel objet d'examen dans le deuxième état, la représentation synthétique (SR) représentant la zone d'examen après l'application d'une quantité de l'agent de contraste, la deuxième quantité étant différente de la première quantité,
- l'unité de commande et de calcul (12) étant configurée pour amener l'unité de sortie (13) à fournir en sortie et/ou à mémoriser la représentation synthétique (SR) et/ou à la transmettre à un système d'ordinateur distinct.

13. Produit programme d'ordinateur comprenant un programme d'ordinateur (40) qui est apte à être chargé dans une mémoire de travail (22) d'un système d'ordinateur (1) et qui, dans celui-ci, amène le système d'ordinateur (1) à mettre en œuvre les étapes suivantes :

- réception d'au moins une représentation d'entrée (R1,2) de la zone d'examen de l'objet d'examen dans un premier état, ladite au moins une représentation d'entrée (R1,2) représentant la zone d'examen avant et/ou après l'application d'une première quantité d'un agent de contraste,
- fourniture d'un modèle entraîné (MLM$^t$) de l'apprentissage automatique,

    • le modèle entraîné (MLM$^t$) d'apprentissage automatique ayant été entraîné à partir de données d'entraînement (TD) de façon à générer, sur la base d'au moins une représentation d'entrée (R1, R2) une zone d'examen d'un objet d'examen dans un premier état, une représentation synthétique (SR) de la zone d'examen dans un deuxième état,
    • les données d'entraînement (TD) comprenant, pour chaque objet d'examen d'une pluralité d'objets d'examen,

        i) une représentation d'entrée (R1) de la zone d'examen,
        ii) une représentation cible (TR) et iii) une représentation cible transformée (TR$^T$),

    • la représentation cible transformée (TR$^T$) représentant au moins une partie de la zone d'examen de l'objet d'examen dans le domaine fréquentiel, si la représentation cible (TR) représente la zone d'examen de l'objet d'examen dans le domaine spatial, ou la représentant dans le domaine spatial, si la représentation cible (TR) représente la zone d'examen de l'objet d'examen dans le domaine fréquentiel,
    • le modèle (MLM$^t$) d'apprentissage automatique ayant été entraîné, pour chaque objet d'examen, de façon à rendre minimaux les écarts entre i) au moins une partie de la représentation synthétique (SR) et au moins une partie de la représentation cible (TR) et ii) entre au moins une partie d'une représentation synthétique transformée (SR$^T$) et au moins une partie de la représentation cible transformée (TR$^T$),

- fourniture en entrée, au modèle entraîné (MLM$^t$) d'apprentissage automatique, de ladite au moins une représentation d'entrée reçue (R1,2) de la zone d'examen de l'objet d'examen,
- réception, en provenance du modèle (MLM$^t$) d'apprentissage automatique, d'une représentation synthétique (SR) de la zone d'examen de l'objet d'examen dans le deuxième état, la représentation synthétique (SR) représentant la zone d'examen après l'application d'une deuxième quantité de l'agent de contraste, la deuxième quantité étant différente de la première quantité,
- fourniture en sortie et/ou mémorisation de la représentation synthétique (SR*) et/ou transmission de la représentation synthétique (SR*) à un système d'ordinateur distinct.

Fig. 1

Fig. 2

Fig. 3

Fig. 4

Fig. 5

Fig. 6

Fig. 7

Fig. 8

Fig. 9

**(a)**

TR  SR  $\Delta$R

**(b)**

TR  SR  $\Delta$R

Fig. 10

**Fig. 11**

Fig. 12

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2019074938 A1
- US 10997716 B2
- WO 2021052896 A1
- WO 2021197996 A1

**In der Beschreibung aufgeführte Nicht-Patentliteratur**

- **R. MECHREZ et al.** The Contextual Loss for Image Transformation with Non-Aligned Data. *arXiv:1803.02077v4*, 2018
- **H. ZHAO et al.** Loss Functions for Image Restoration with Neural Networks. *arxiv:1511.08861v3*, 2018
- U-net: Convolutional networks for biomedical image segmentation. **O. RONNEBERGER et al.** International Conference on Medical image computing and computer-assisted intervention. Springer, 2015, 234-241
- **M.-Y. LIU et al.** Generative Adversarial Networks for Image and Video Synthesis: Algorithms and Applications. *arXiv:2008.02793*
- **J. HENRY et al.** *Pix2Pix GAN for Image-to-Image Translation*
- **Q. LI et al.** RegGAN: An End-to-End Network for Building Footprint Generation with Boundary Regularization. *Remote Sens.*, 2022, vol. 14, 1835
- **P. ISOLA et al.** Image-to-Image Translation with Conditional Adversarial Networks. *arXiv:1611.07004 [cs.CV*
- **D. KARIMI et al.** Convolution-Free Medical Image Segmentation using Transformers. *arXiv:2102.13645 [eess.IV*